(19) Europäisches Patentamt
European Patent Office
Office européen des brevets

(11) Veröffentlichungsnummer: **0 338 988 A2**

(12) **EUROPÄISCHE PATENTANMELDUNG**

(21) Anmeldenummer: **89810281.9**

(22) Anmeldetag: **11.04.89**

(51) Int. Cl.[4]: **C 07 C 157/12**
A 01 N 47/34, C 07 C 157/14

(30) Priorität: **20.04.88 CH 1450/88**
**05.05.88 CH 1690/88**

(43) Veröffentlichungstag der Anmeldung:
**25.10.89 Patentblatt 89/43**

(84) Benannte Vertragsstaaten:
**AT BE CH DE ES FR GB IT LI NL**

(71) Anmelder: **CIBA-GEIGY AG**
**Klybeckstrasse 141**
**CH-4002 Basel (CH)**

(72) Erfinder: **Drabek, Jozef, Dr.**
**Benkenstrasse 12**
**CH-4104 Oberwil (CH)**

**Böger, Manfred**
**Wilhelm Glockstrasse 14**
**D-7858 Weil am Rhein 5 (DE)**

**Ehrenfreund, Josef, Dr.**
**Amselstrasse 11**
**CH-4123 Allschwil (CH)**

Patentanspruch für folgenden Vertragsstaat: ES.

(54) **Thioharnstoffe.**

(57) Neue N-Phenyl-N-carbonyl-thioharnstoffe der Formel I

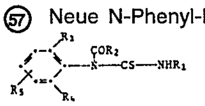

(I),

worin

$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein-oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,

$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes Phenyl oder $C_3$-$C_8$-Cycloalkyl; oder für einen Rest -$OR_9$;

$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;

$R_4$ für $C_1$-$C_{10}$-Alkyl, $C_3$-$C_7$-Cycloalkyl oder $C_5$-$C_6$-Cycloalkenyl;

$R_5$ für Wasserstoff oder $R_6(Y)_n$;

$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;

$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;

Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und

n für 0 oder 1

stehen, mit der Massgabe, dass $R_2$ nur dann für den Rest -$OR_9$ stehen darf, wenn $R_5$ eine von Wasserstoff und $C_1$-$C_{10}$-Alkoxy verschiedene Bedeutung hat; Verfahren und Salze entsprechender Isothioharnstoffe als Zwischenprodukte zu ihrer Herstellung, ihre Verwendung in der Schädlingsbekämpfung, sowie Schädlingsbekämpfungsmittel, welche mindestens eine Verbindung der Formel I oder ein aktives Zwischenprodukt enthalten, werden offenbart. Bevorzugtes Anwendungsgebiet ist die Bekämpfung von Schädlingen an Tieren und Pflanzen.

EP 0 338 988 A2

**Beschreibung**

## Thioharnstoffe

Die vorliegende Erfindung betrifft neue substituierte N-Phenyl-N-acyl-thioharnstoffe und N-Phenyl-N-carboxyl-thioharnstoffe, Verfahren und Zwischenprodukte zu ihrer Herstellung, Schädlingsbekämpfungsmittel, welche diese Verbindungen enthalten, sowie ihre Verwendung bei der Kontrolle von Schädlingen.

Die erfindungsgemässen Verbindungen entsprechen der Formel I

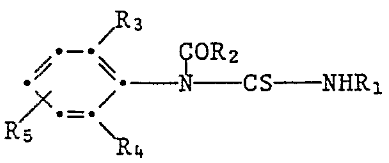

worin

$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein-oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,

$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes Phenyl oder $C_3$-$C_8$-Cycloalkyl; oder für einen Rest -$OR_9$;

$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;

$R_4$ für $C_1$-$C_{10}$-Alkyl, $C_3$-$C_7$-Cycloalkyl oder $C_5$-$C_6$-Cycloalkenyl;

$R_5$ für Wasserstoff oder $R_6(Y)_n$;

$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;

$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;

Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und

n für 0 oder 1

stehen, mit der Massgabe, dass $R_2$ nur dann für den Rest -$OR_9$ stehen darf, wenn $R_5$ eine von Wasserstoff und $C_1$-$C_{10}$-Alkoxy verschiedene Bedeutung hat.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden Alkyl- und Alkoxyreste können geradkettig oder verzweigt sein. Als Beispiele solcher Alkyle seien u.a. Methyl, Aethyl, Propyl, Isopropyl, Butyl, i-Butyl, sek.Butyl, tert.Butyl oder Pentyl, Hexyl, Octyl und ihre Isomeren genannt. Als geeignete Alkoxyreste seien u.a. genannt: Methoxy, Aethoxy, Propoxy, Isopropoxy, oder Butoxy und ihre Isomeren.

Die als Substituenten in Betracht kommenden Alkenyle und Alkinyle können geradkettig oder verzweigt sein und eine oder mehrere ungesättigte Bindungen enthalten. Dabei ist es auch möglich, dass zwei- und dreifach ungesättigte Bindungen im gleichen Rest auftreten können. Vorzugsweise enthalten diese ungesättigten Reste zwei bis sechs Kohlenstoffatome. Beispiele solcher Alkenyle und Alkinyle sind u.a. Vinyl, Allyl, 1-Propenyl, Isopropenyl, Allenyl, Butenyle, Butadienyle, Hexenyle, Hexandienyl, Aethinyl, 1-Propinyl, 2-Propinyl, Butinyle, Pentinyle, Hexinyle, Hexadiinyle, 2-Penten-4-inyl.

Die als Substituenten in Betracht kommenden Phenylalkyle können geradkettig oder verzweigt sein. Geeignete Beispiele sind u.a. Benzyl, Phenäthyl, Phenpropyl, Phenisopropyl, Phenbutyl und seine Isomeren.

Sind die als Substituenten in Betracht kommenden Alkyle, Alkoxyle, Alkenyle, Alkinyle oder Phenylalkyle durch Halogen substituiert, so können sie nur teilweise oder auch perhalogeniert sein. Die Halogensubstitution der Phenylalkyle kann sowohl im Phenylkern als auch in der Alkylkette oder in beiden gleichzeitig erfolgen. Dabei gelten für die Halogene, Alkyle, Alkoxyle, Alkenyle, Alkinyle und Phenylalkyle die oben gegebenen Definitionen. Beispiele solcher Gruppen sind u.a. das ein- bis dreifach durch Fluor, Chlor und/oder Brom substituierte Methyl wie z.B. $CHF_2$ oder $CF_3$; das ein- bis fünffach durch Fluor, Chlor und/oder Brom substituierte Aethyl wie z.B. $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein-bis siebenfach durch Fluor, Chlor und/oder Brom substituierte Propyl oder Isopropyl wie z.B. $CH_2CHBrCH_2Br$, $CF_2CHFCF_3$, $CH_2CF_2CF_3$, oder $CH(CF_3)_2$; das ein- bis neunfach durch Fluor, Chlor und/oder Brom substituierte Butyl oder eines seiner Isomeren wie z.B. $CF(CF_3)CHFCF_3$ oder $CH_2(CF_2)_2CF_3$; das ein- bis dreifach durch Fluor, Chlor und/oder Brom substituierte Vinyl, Propinyl oder Pentadiinyl; das ein- bis fünffach durch Fluor, Chlor und/oder Brom substituierte Allyl,

1-Propenyl, Butadienyl oder ein Butinyl; ein ein- bis siebenfach durch Fluor, Chlor und/oder Brom substituiertes Butenyl, Pentadienyl oder Pentinyl; das ein- bis siebenfach durch Fluor, Chlor und/oder Brom substituierte Benzyl; das ein- bis neunfach durch Fluor, Chlor und/oder Brom substituierte Phenäthyl; das ein- bis elffach durch Fluor, Chlor und/oder Brom substituierte Phenpropyl oder Phenisopropyl.

Bei den als Substituenten in Betracht kommenden Alkylen, Alkenylen, Alkinylen und Phenylalkylen, die durch Sauerstoff und/oder Schwefel unterbrochen sind, kann es sich sowohl um einfache Aether als auch um Polyalkylenglykole oder -thioglykole handeln. Dabei sind auch Ketten möglich, die Sauerstoff und Schwefel gleichzeitig enthalten. Beispiele sind u.a. Reste wie Methoxymethyl, Methylthiomethyl, Methoxymethoxymethyl, Methoxyäthyl, Aethoxyäthyl, Aethylthioäthyl, Vinyloxymethyl, Allylthioäthyl, Methoxypropinyl, Benzyloxymethyl, Benzylthioäthyl.

Bei den als Substituenten in Betracht kommenden Cycloalkylen und Cycloalkenylen handelt es sich beispielsweise um Cyclopropyl, Cyclobutyl, Cyclopentyl, 2-Cyclopenten-1-yl, Cyclohexyl oder Cyclohexenyl. Diese Reste können ein- oder mehrfach durch Halogen oder Alkyl substituiert sein wie z.B. 2-Chlorcyclopropyl, 2,2-Dimethylcyclopropyl, 2-Chlorcyclobutyl, 2-Methylcyclohexyl, 2,4-Dimethylcyclohexyl, 2,4,6-Trimethylcyclohexyl und/oder über eine $C_1$-$C_4$-Alkylenbrücke mit dem Rest des Moleküls verbunden sein, wie z.B. 1-Cyclopropyläthyl.

Unter den Verbindungen der Formel I sind zwei Untergruppen herauszuheben. Eine entspricht der Formel Ia

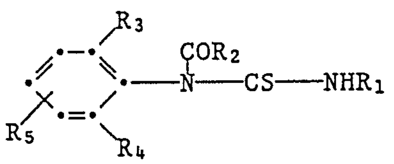 (Ia),

worin
$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl;
$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl;
$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;
$R_4$ für $C_1$-$C_{10}$-Alkyl, $C_3$-$C_7$-Cycloalkyl oder $C_5$-$C_6$-Cycloalkenyl;
$R_5$ für Wasserstoff oder $R_6(Y)_n$;
$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und
n für 0 oder 1
stehen.

Die zweite wird unter der Formel Ib

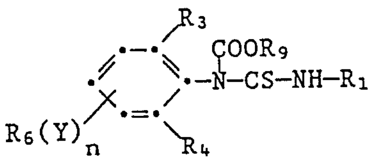 (Ib),

zusammengefasst, worin
$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein-oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,
$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;
$R_4$ für $C_1$-$C_{10}$-Alkyl, $C_3$-$C_7$-Cycloalkyl oder $C_5$-$C_6$-Cycloalkenyl;
$R_6$ für Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;
$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl

bedeuten; und
n für 0 oder 1
stehen.

Unter den Verbindungen der Formel Ia stehen diejenigen im Vordergrund, in denen $R_1$ für $C_1$-$C_5$-Alkyl oder Phenyl-$C_1$-$C_3$-alkyl; $R_2$ für $C_1$-$C_5$-Alkyl, $C_2$-$C_5$-Alkenyl, $C_2$-$C_5$-Alkinyl, Phenyl oder Phenyl-$C_1$-$C_3$alkyl; $R_3$ für $C_1$-$C_5$-Alkyl, $C_1$-$C_5$-Alkoxy, Cyclopentyl oder Cyclopentenyl; $R_4$ für $C_1$-$C_5$-Alkyl; $R_5$ für Wasserstoff oder $R_6(Y)_n$; $R_6$ für $C_1$-$C_5$-Alkyl, Phenyl, Naphthyl oder Indanyl; oder ein- oder mehrfach durch Fluor, Chlor oder $C_1$-$C_3$-Alkyl substituiertes Phenyl, Naphthyl oder Indanyl; Y für O, S, $SO_2$, NH, N(CHO), $N(CH_3)$ oder $C(R_7)R_8$, wobei $R_7$ und $R_8$ je Wasserstoff oder Methyl bedeuten; und n für 0 oder 1 stehen.

Dabei sind diejenigen Verbindungen der Formel Ia bevorzugt, in denen $R_1$ für $C_3$-$C_5$-Alkyl oder Phenyl-$C_2$-$C_3$-alkyl; $R_2$ für $C_1$-$C_3$-Alkyl oder Phenyl; $R_3$ für $C_1$-$C_4$-Alkyl, $C_1$-$C_4$-Alkoxy, Cyclopentyl oder Cyclopentenyl; $R_4$ für $C_1$-$C_4$-Alkyl; $R_5$ für Wasserstoff oder $R_6Y$ in 4-Stellung; $R_6$ für $C_1$-$C_4$-Alkyl, Phenyl oder durch 1 bis 2 Fluor substituiertes Phenyl; und Y für O oder S stehen.

Unter den Verbindungen der Formel Ib stehen diejenigen im Vordergrund, in denen $R_1$ für $C_1$-$C_5$-Alkyl oder Phenyl-$C_1$-$C_3$-alkyl; $R_3$ für $C_1$-$C_5$-Alkyl, $C_1$-$C_5$-Alkoxy, Cyclopentyl oder Cyclopentenyl; $R_4$ für $C_1$-$C_5$-Alkyl; $R_6$ für Phenyl, Naphthyl oder Indanyl; $R_9$ für $C_1$-$C_5$-Alkyl, $C_3$-$C_5$-Alkenyl, $C_3$-$C_5$-Alkinyl, Phenyl oder Phenyl-$C_1$-$C_3$alkyl; Y für O, S, $SO_2$, NH, N(CHO), $N(CH_3)$ oder $C(R_7)R_8$, wobei $R_7$ und $R_8$ je Wasserstoff oder Methyl bedeuten; und n für 0 oder 1 stehen.

Dabei sind diejenigen Verbindungen der Formel Ib bevorzugt, in denen $R_1$ für $C_3$-$C_5$-Alkyl oder Phenyl-$C_2$-$C_3$-alkyl; $R_3$ für $C_1$-$C_3$-Alkyl, $C_1$-$C_4$-Alkoxy, Cyclopentyl; $R_4$ für $C_1$-$C_4$-Alkyl; $R_6$ für Phenyl oder durch 1 bis 2 Fluor substituiertes Phenyl; $R_9$ für $C_1$-$C_3$-Alkyl oder Phenyl; Y in 4-Stellung für O oder S; und n für 1 stehen.

Die erfindungsgemässen Verbindungen der Formel I können nach im Prinzip bekannten Verfahren hergestellt werden, indem man z.B.

a) einen Thioharnstoff der Formel II

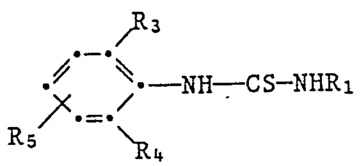

(II)

mit einem Acylhalogenid der Formel III
$XCOR_2$ (III)
in einem Lösungsmittel und in Gegenwart einer Base unter normalem Druck bei -30 bis +100°C umsetzt oder

b) ein Salz eines Isothioharnstoffes der Formel IV

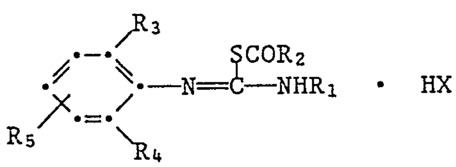

(IV)

bei Raumtemperatur in einer wässrigen Lösung hydrolisiert. In den Formeln II bis IV haben $R_1$, $R_2$, $R_3$, $R_4$ und $R_5$ die für Formel I angegebene Bedeutung und X steht für den Rest einer Halogenwasserstoffsäure, insbesondere für Chlor.

Als Lösungsmittel eignen sich gegenüber den Reaktionsteilnehmern inerte Lösungs- und Verdünnungsmittel wie z.B. Aether oder ätherartige Verbindungen wie u.a. Diäthyläther, Di-isopropyläther, Dioxan oder Tetrahydrofuran; aromatische Kohlenwasserstoffe wie Benzol, Toluol oder Xylole; Ketone wie Aceton, Methyläthylketon oder Cyclohexanon; Nitrile wie Acetonitril; Halogenkohlenwasserstoffe wie Chloroform oder Methylenchlorid; oder Dimethylformamid.

Geeignete Basen können organischen oder anorganischen Ursprungs sein wie z.B. Natriumhydrid, Natrium-, Kalium- oder Calciumcarbonat, tertiäre Amine wie Triäthylamin, Triäthylendiamin oder 4-Dimethylaminopyridin oder Pyridin.

Die Thioharnstoffe der Formel II ihrerseits können nach im Prinzip bekannten Verfahren hergestellt werden, z.B. indem man ein Isothiocyanat der Formel V

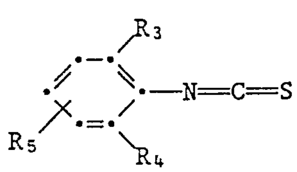

(V)

mit einem Amin der Formel VI

$H_2N-R_1$ (VI)
umsetzt, wobei in den Formeln V und VI $R_1$, $R_3$, $R_4$ und $R_5$ die für Formel I angegebenen Bedeutungen haben.

Das Verfahren zur Herstellung der Verbindungen der Formel II wird zweckmässigerweise in Gegenwart eines gegenüber den Reaktionsteilnehmern inerten Lösungs- oder Verdünnungsmittels, bei einer Reaktionstemperatur von 0 bis +100°C unter normalem Druck durchgeführt. Geeignete Lösungs-und Verdünnungsmittel sind beispielsweise schon für das Verfahren zur Herstellung der Verbindungen der Formel I aufgezählt worden.

Die Salze der Isothioharnstoffe der Formel IV können nach im Prinzip bekannten Verfahren hergestellt werden, z.B. indem man einen Thioharnstoff der Formel II mit einem Acylhalogenid der Formel III in einem Lösungsmittel unter normalem Druck bei einer Temperatur von -20° bis +100°C umsetzt. Dabei ist die Umsetzung mit einem Acylchlorid besonders bevorzugt.

Die Verbindungen der Formel IV sind neu und bilden als Zwischenprodukte ebenfalls einen Gegenstand der vorliegenden Erfindung. Die Verbindungen der Formeln II, III, V und VI dagegen sind bekannt und können nach im Prinzip bekannten Verfahren hergestellt werden.

Es wurde nun gefunden, dass die erfindungsgemässen Verbindungen der Formel I, sowie die Zwischenprodukte der Formel IV bei günstiger Warmblüter-, Fisch- und Pflanzenverträglichkeit wertvolle Wirkstoffe in der Schädlingsbekämpfung sind. So eignen sich die Verbindungen der Formeln I und IV z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können Larven und Eier von phytopathogenen Schadinsekten und -milben in Zier- und Nutzpflanzungen, wie z.B. in Obst- und Gemüsepflanzungen, aber auch saugende Insekten, wie z.B. Reiszikaden und Aphiden, und Bodeninsekten, wie z.B. Diabrotica-Arten in Maiskulturen, wirkungsvoll bekämpft werden. Werden Verbindungen der Formeln I und IV von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus-und Nutztieren, kommen vor allem Ektoparasiten, wie z.B. Milben und Zecken und Dipteren, wie z.B. Lucilia sericata in Betracht. Ausserdem weisen die Verbindungen der Formel I und ihre Zwischenprodukte der Formel IV eine beachtliche mikrobizide Wirkung auf. Dabei können vor allem pflanzenpathogene Pilze der Klasse Oomycetidae, wie z.B. Familien der Ordnung Peronosporales (Plasmopara sp.) nachhaltig unter Kontrolle gebracht werden.

Unter den Verbindungen der Formel IV sind als hervorzuhebende Untergruppe diejenigen der Formel IVa

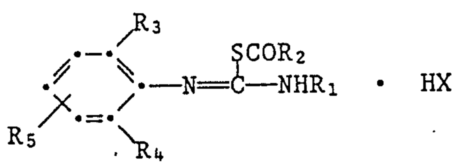

(IVa)

zu nennen, worin
$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl;
$R_2$ für $C_1$-$C_{10}$-Alkyl, $C_2$-$C_{10}$-Akenyl, $C_2$-$C_{10}$-Alkinyl, Phenyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl;
$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;
$R_4$ für $C_1$-$C_{10}$-Alkyl, $C_3$-$C_7$-Cycloalkyl oder $C_5$-$C_6$-Cycloalkenyl;
$R_5$ für Wasserstoff oder $R_6(Y)_n$;
$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und n für 0 oder 1 stehen, und
X den Rest einer Halogenwasserstoffsäure bedeutet.

Von den Verbindungen der Formel VIa sind als bevorzugt diejenigen zu nennen, worin $R_1$ für $C_1$-$C_5$-Alkyl oder Phenyl-$C_1$-$C_3$-alkyl; $R_2$ für $C_1$-$C_5$-Alkyl, $C_2$-$C_5$-Alkenyl, $C_2$-$C_5$-Alkinyl, Phenyl oder Phenyl-$C_1$-$C_3$-alkyl; $R_3$ für $C_1$-$C_5$-Alkyl, $C_1$-$C_5$-Alkoxy, Cyclopentyl oder Cyclopentenyl; $R_4$ für $C_1$-$C_5$-Alkyl; $R_5$ für Wasserstoff oder $R_6(Y)_n$; $R_6$ für $C_1$-$C_5$-Alkyl, Phenyl, Naphthyl oder Indanyl; oder ein- oder mehrfach durch Fluor, Chlor oder $C_1$-$C_3$-Alkyl substituiertes Phenyl, Naphthyl oder Indanyl; Y für O, S, $SO_2$, NH, N(CHO), $N(CH_3)$ oder $C(R_7)R_8$, wobei $R_7$ und $R_8$ je Wasserstoff oder Methyl bedeuten; und n für 0 oder 1 stehen; und X den Rest einer Halogenwasserstoffsäure bedeutet.

Ganz besonders heben sich aber die Verbindungen der Formel IVa ab, worin $R_1$ für $C_3$-$C_5$-Alkyl oder Phenyl-$C_2$-$C_3$-alkyl; $R_2$ für $C_1$-$C_3$-Alkyl oder Phenyl; $R_3$ für $C_1$-$C_4$-Alkyl, $C_1$-$C_4$-Alkoxy, Cyclopentyl oder Cyclopentenyl; $R_4$ für $C_1$-$C_4$-Alkyl; $R_5$ für Wasserstoff oder $R_6$Y in 4-Stellung; $R_6$ für $C_1$-$C_4$-Alkyl, Phenyl oder durch 1 oder 2 Fluor substituiertes Phenyl; und Y für O oder S stehen; und X Chlor bedeutet.

Die gute pestizide Wirkung der erfindungsgemässen Verbindungen der Formeln I und IV entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formeln I und IV werden in unveränderter Form oder vorzugsweise zusammen mit den in der Formulierungstechnik üblichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formeln I und/oder IV, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formeln I oder IV oder der Kombinationen dieser Wirkstoffe mit andern Insektiziden oder Akariziden nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 2 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"1985 International Mc Cutcheon's Emulsifiers & Detergents", Glen Rock NJ USA, 1985",
"H. Stache, "Tensid-Taschenbuch", 2. Aufl., C. Hanser Verlag München, Wien 1981,
M. and J. Ash. "Encyclopedia of Surfactants", Vol. I-III, Chemical Publishing Co., New York, 1980-1981.

Die pestiziden Zubereitungen enthalten in der Regel 0,1 bis 99 %, insbesondere 0,1 bis 95 %, (+)-Enantiomer des Wirkstoffs der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen: (% = Gewichtsprozent)

| | | | |
|---|---|---|---|
| Emulgierbare Konzentrate | | | |
| Aktiver Wirkstoff: | 1 bis 20 %, | bevorzugt | 5 bis 10 % |
| oberflächenaktives Mittel: | 5 bis 30 %, | vorzugsweise | 10 bis 20 % |
| flüssiges Trägermittel: | 50 bis 94 %, | vorzugsweise | 70 bis 85 % |
| Stäube: | | | |
| Aktiver Wirkstoff: | 0,1 bis 10 %, | vorzugsweise | 0,1 bis 1 % |
| festes Trägermittel: | 99,9 bis 90 %, | vorzugsweise | 99,9 bis 99 % |
| Suspension-Konzentrate | | | |
| Aktiver Wirkstoff: | 5 bis 75 %, | vorzugsweise | 10 bis 50 % |
| Wasser: | 94 bis 24 %, | vorzugsweise | 88 bis 30 % |
| oberflächenaktives Mittel: | 1 bis 40 %, | vorzugsweise | 2 bis 30 % |
| Benetzbare Pulver | | | |
| Aktiver Wirkstoff: | 0,5 bis 90 %, | vorzugsweise | 1 bis 80 % |
| oberflächenaktives Mittel: | 0,5 bis 20 %, | vorzugsweise | 1 bis 15 % |
| festes Trägermaterial: | 5 bis 95 %, | vorzugsweise | 15 bis 90 % |
| Granulate | | | |
| aktiver Wirkstoff: | 0,5 bis 30 %, | vorzugsweise | 3 bis 15 % |
| festes Trägermittel: | 99,5 bis 70 %, | vorzugsweise | 97 bis 85 % |

Die Mittel können auch weitere Zusätze wie Stabilisatoren, Entschäumer, Konservierungmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

## Beispiel 1: Herstellung

### 1.1. Zwischenprodukte

#### 1.1.1. N-[2,6-Diisopropyl-4-(2-fluorphenoxy)-phenyl]-N'-isopropyl-S-acetyl-isothioharnstoff Hydrochlorid

7,7 g N-[2,6-Diisopropyl-4-(2-fluorphenoxy)-phenyl]-N'-isopropyl-thioharnstoff werden in 20 ml Aceton gelöst und unter Rühren tropfenweise bei +5 bis 10°C mit 2,36 g Acetylchlorid versetzt. Das Reaktionsgemisch wird anschliessend 3 Stunden lang bei +10°C stehen gelassen. Das auskristallisierte Produkt wird abgenutscht, mit wenig Aceton gewaschen und am Vakuum getrocknet. Die Titelverbindung der Formel

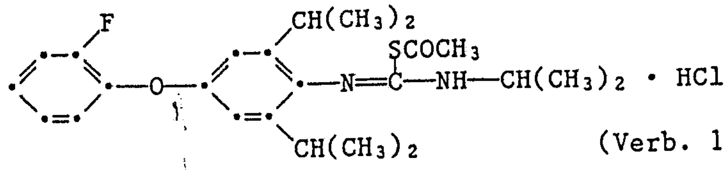

(Verb. 1.1.1.)

liege in Form farbloser Kristalle vor; Smp. 164°C unter Zersetzung.

Auf analoge Weise werden die folgenden Verbindungen hergestellt:

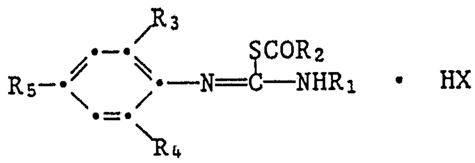

| Verb. Nr. | $R_1$ | $R_2$ | $R_3$ | $R_4$ | $R_5$ | X | Smp. C° |
|---|---|---|---|---|---|---|---|
| 1.1.2. | $C(CH_3)_3$ | $CH_3$ | $C_2H_5$ | $CH(CH_3)_2$ | H | Cl | 124-128 |
| 1.1.3. | $CH(CH_3)_2$ | $CH_3$ | $C_2H_5$ | $CH(CH_3)_2$ | H | Cl | 159-161 |
| 1.1.4. | $C(CH_3)_3$ | 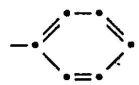 | $C_2H_5$ | $C_2H_5$ | H | Cl | 130 |
| | $C(CH_3)_3$ | $CH_3$ | $CH(CH_3)_2$ | $CH(CH_3)_2$ | H | Cl | |
| | $C(CH_3)_3$ | $CH_3$ | $CH(CH_3)_2$ | $CH(CH_3)_2$ | 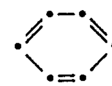—O— | Cl | |
| | $CH(CH_3)_2$ | $CH(CH_3)_2$ | $C_2H_5$ | $CH(CH_3)_2$ | H | Cl | |
| | $CH(CH_3)_2$ | 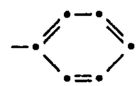 | $C_2H_5$ | $CH(CH_3)_2$ | H | Cl | |
| | $CH(CH_3)_2$ | $CH_3$ | $CH(CH_3)_2$ | $CH(CH_3)_2$ | 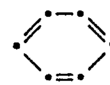—$N(CH_3)$— | Cl | |
| | $CH(CH_3)_2$ | $CH_3$ | $CH(CH_3)_2$ | $CH(CH_3)_2$ | $CH_3S$— | Cl | |
| | $CH(CH_3)_2$ | $CH_3$ | $CH(CH_3)_2$ | $CH(CH_3)_2$ | $CH_3SO_2$— | Cl | |

| Verb. Nr. | $R_1$ | $R_2$ | $R_3$ | $R_4$ | $R_5$ | X | Smp. C° |
|---|---|---|---|---|---|---|---|
| | $CH(CH_3)_2$ | $CH_3$ | $CH(CH_3)_2$ | $CH(CH_3)_2$ | 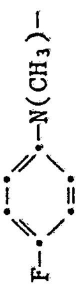 | Cl | |
| | $CH(CH_3)_2$ | $CH_3$ | Cyclopentyl | $CH(CH_3)_2$ | H | Cl | |
| | $CH(CH_3)_2$ | $CH_3$ | Cyclopentyl | $C_2H_5$ | H | Cl | |
| | $CH(CH_3)_3$ | $CH(CH_3)_2$ | Cyclopentyl | $CH(CH_3)_2$ | H | Cl | |
| | $CH(CH_3)_2$ | $CH_3$ | $CH(CH_3)_2$ | $CH(CH_3)_2$ | 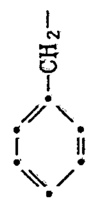 | Cl | |

1.2. Endprodukte

1.2.1. N-(2-Aethyl-6-isopropylphenyl)-N-acetyl-N'-tert.butylthioharnstoff

9,75 g N-(2-Aethyl-6-isopropylphenyl)-N'-tert.butyl-thioharnstoff, 3,55 g Triäthylamin und 0,2 g 4-Dimethylaminopyridin werden in 50 ml Aceton gelöst und unter Rühren tropfenweise bei 0 bis +5°C mit 2,75 g Acetylchlorid versetzt. Anschliessend wird das Reaktionsgemisch eine Stunde lang bei 0 bis +5°C weitergerührt, dann filtriert und der Rückstand mit Aceton gewaschen. Das Aceton wird durch Destillation aus dem Filtrat entfernt und der entstandene Rückstand an Silicagel mit einer Hexan/Methylenchlorid-Mischung

chromatographisch gereinigt. Die Titelverbindung der Formel

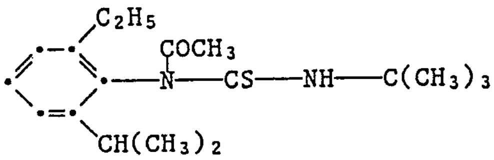

(Verb. 1.2.1.)

liegt in Form eines blassgelben Oels vor; Brechungsindex $n_D^{24}$ : 1,5379.

1.2.2. N-[2,6-Diisopropyl-4-(2-fluorphenoxy)-phenyl]-N-acetyl-N'-isopropyl-thioharnstoff

3,1 g N-[2,6-Diisopropyl-4-(2-fluorphenoxy)-phenyl]-N'-isopropyl-S-acetyl-isothioharnstoff Hydrochlorid werden in 60 ml Aceton und 10 ml Wasser suspendiert und 24 Stunden lang bei Raumtemperatur gerührt. Anschliessend wird das Raktionsgemisch eingeengt und der Rückstand aus Hexan umkristallisiert. Die Titelverbindung der Formel

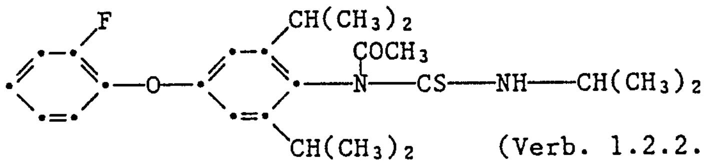

(Verb. 1.2.2.)

liegt in Form von farblosen Kristallen vor; Smp. 122-126°C.

Auf analoge Weise werden die folgenden Verbindungen hergestellt:

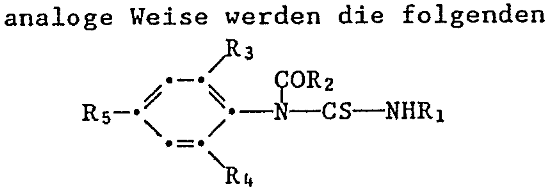

| Verb. Nr. | $R_1$ | $R_2$ | $R_3$ | $R_4$ | $R_5$ | phys. Daten |
|---|---|---|---|---|---|---|
| 1.2.3. | $CH(CH_3)_2$ | $CH_3$ | $CH(CH_3)_2$ | $CH(CH_3)_2$ | H | Smp. 85-88°C |
| 1.2.4. | $CH(CH_3)_2$ | $CH_3$ | $C_2H_5$ | $CH(CH_3)_2$ | H | $n_D^{24}$: 1,5492 |
| 1.2.5. | $CH(CH_3)_2$ | $CH_3$ | $C_2H_5$ | $CH(CH_3)C_2H_5$ | 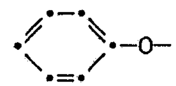 | $n_D^{24}$: 1,5632 |
| 1.2.6. | $CH(CH_3)_2$ | $CH(CH_3)_2$ | $C_2H_5$ | $CH(CH_3)_2$ | H | $n_D^{25}$: 1,5440 |
| 1.2.7. | $CH(CH_3)_2$ | $C_2H_5$ | $C_2H_5$ | $CH(CH_3)_2$ | H | Smp. 69-71°C |
| 1.2.8. | $CH(CH_3)_2$ | 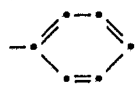 | $C_2H_5$ | $CH(CH_3)_2$ | H | Smp. 78-80°C |
| 1.2.9. | $CH(CH_3)_2$ | $CH_3$ | $C_2H_5$ | $CH(CH_3)_2$ | 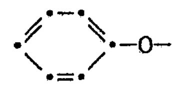 | Smp. 61-63°C |
| 1.2.10. | $CH(CH_3)_2$ | $CH_3$ | $C_2H_5$ | $CH(CH_3)C_2H_5$ | H | $n_D^{24}$: 1,5459 |
| 1.2.11. | $CH(CH_3)_2$ | $CH_3$ | $C_2H_5$ | $C_2H_5$ | H | $n_D^{23}$: 1,5562 |
| 1.2.12. | $CH(CH_3)_2$ | 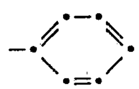 | $C_2H_5$ | $C_2H_5$ | H | Smp. 60-62°C |
| 1.2.13. | $CH(CH_3)C_2H_5$ | $CH_3$ | $C_2H_5$ | $CH(CH_3)_2$ | H | $n_D^{23}$: 1,5452 |

Fortsetzung Tabelle

| Verb. Nr. | $R_1$ | $R_2$ | $R_3$ | $R_4$ | $R_5$ | phys. Daten |
|---|---|---|---|---|---|---|
| 1.2.14. | $CH(CH_3)_2$ | $CH_3$ | $CH(CH_3)_2$ | $CH(CH_3)_2$ | 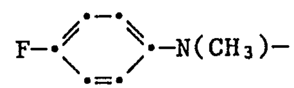 | Smp. 124-126°C |
| 1.2.15. | $CH(CH_3)_2$ | $CH_3$ | $CH(CH_3)_2$ | $CH(CH_3)_2$ | $(CH_3)_2CH{-}S{-}$ | Smp. 84-86°C |
| 1.2.16. | $CH(CH_3)_2$ | $CH_3$ | $CH(CH_3)_2$ | $CH(CH_3)_2$ | 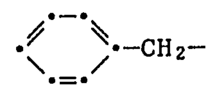 | Smp. 83,5-85°C |

| Verb. Nr. | $R_1$ | $R_2$ | $R_3$ | $R_4$ | $R_5$ | phys. Daten |
|---|---|---|---|---|---|---|
| | $C(CH_3)_3$ | $CH_3$ | $CH(CH_3)_2$ | $CH(CH_3)_2$ | 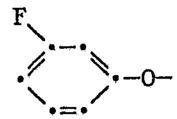 | |
| | $C(CH_3)_3$ | $CH_3$ | $CH(CH_3)_2$ | $CH(CH_3)_2$ | H | |
| | $C(CH_3)_3$ | $CH_3$ | $CH(CH_3)_2$ | $CH(CH_3)_2$ | 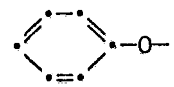 | |
| | $C(CH_3)_3$ | $CH_3$ | $C_2H_5$ | $CH(CH_3)C_2H_5$ | H | |
| | $C(CH_3)_3$ | $CH_3$ | $CH_3$ | $CH(CH_3)_2$ | H | |
| | $C(CH_3)_3$ | $CH_3$ | $CH_3$ | $C(CH_3)_3$ | H | |
| | $C(CH_3)_3$ | $CH_3$ | $CH(CH_3)_2$ | $CH(CH_3)_2$ | 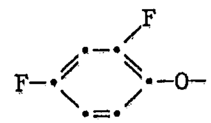 | |
| | $-C(CH_3)_2-$ 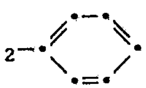 | $CH_3$ | $C_2H_5$ | $CH(CH_3)_2$ | H | |
| | $CH(CH_3)_2$ | $-CH_2-CH=CH_2$ | $C_2H_5$ | $CH(CH_3)_2$ | H | |
| | $CH(CH_3)_2$ | $-CH_2-$ 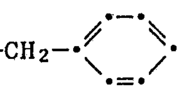 | $C_2H_5$ | $CH(CH_3)_2$ | H | |
| | $CH(CH_3)_2$ | $-(CH_2)_4CH_3$ | $C_2H_5$ | $CH(CH_3)_2$ | H | |
| | $CH(CH_3)_2$ | $CH_3$ | $CH(CH_3)_2$ | $CH(CH_3)_2$ | 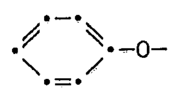 | |

| Verb. Nr. | $R_1$ | $R_2$ | $R_3$ | $R_4$ | $R_5$ | phys. Daten |
|---|---|---|---|---|---|---|
| | $CH(CH_3)_2$ | $CH_3$ | $CH_3$ | $CH(CH_3)_2$ | H | |
| | $CH(CH_3)_2$ | $CH_3$ | $C_2H_5$ | $CH(CH_3)_2$ | 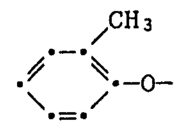 | |
| | $CH(CH_3)_2$ | $CH_3$ | $CH(CH_3)_2$ | $CH(CH_3)_2$ | 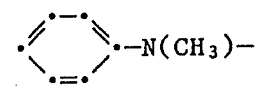 | |
| | $CH(CH_3)_2$ | $CH_3$ | $OCH(CH_3)_2$ | $CH(CH_3)_2$ | $CH(CH_3)_2$ | |
| | $CH(CH_3)_2$ | $CH_3$ | $CH(CH_3)_2$ | $CH(CH_3)_2$ | $CH_3S-$ | |
| | $CH(CH_3)_2$ | $CH_3$ | $CH(CH_3)_2$ | $CH(CH_3)_2$ | 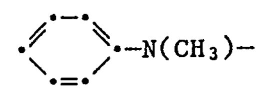 | |
| | $CH(CH_3)_2$ | $CH_3$ | $CH(CH_3)_2$ | $CH(CH_3)_2$ | $CH_3SO_2-$ | |
| | $CH(CH_3)_2$ | $CH_3$ | $OCH(CH_3)_2$ | $CH_3$ | $CH_3$ | |
| | $CH(CH_3)_2$ | $CH_3$ | $CH(CH_3)_2$ | $CH(CH_3)_2$ | $(CH_3)_2CHO-$ | |

| Verb. Nr. | $R_1$ | $R_2$ | $R_3$ | $R_4$ | $R_5$ | phys. Daten |
|---|---|---|---|---|---|---|
| | $CH(CH_3)_2$ | $CH_3$ | $CH(CH_3)_2$ | $CH(CH_3)_2$ | 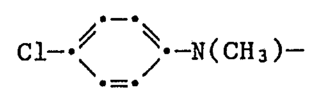 | |
| | $CH(CH_3)_2$ | $CH_3$ | $CH(CH_3)_2$ | $CH(CH_3)_2$ | 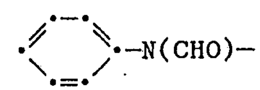 | |
| | $CH(CH_3)_2$ | $CH_3$ | 2-Cyclopentenyl | $CH(CH_3)_2$ | H | |
| | $CH(CH_3)_2$ | $CH_3$ | $CH(CH_3)_2$ | $CH(CH_3)_2$ | $CH(CH_3)_2$ | |
| | $CH(CH_3)_2$ | $CH_3$ | Cyclopentyl | $CH(CH_3)_2$ | 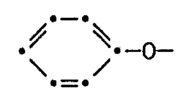 | |
| | $CH(CH_3)_2$ | $CH_3$ | Cyclopentyl | $CH(CH_3)_2$ | H | |
| | $CH(CH_3)_2$ | $CH_3$ | $CH(CH_3)_2$ | $CH(CH_3)_2$ | $CH_3$ | |

1.3.1. N-(2,6-Diisopropyl-4-phenoxyphenyl)-N-äthoxycarbonyl-N′-isopropyl-thioharnstoff

13 g N-(2,6-Diisopropyl)-4-phenoxyphenyl)-N′-isopropyl-thioharnstoff, 6,4 ml Triäthylamin und 0,1 g 4-Dimethylaminopyridin werden in 80 ml Aceton gelöst und unter Rühren tropfenweise bei 0 bis +5°C mit 6,5 g Chlorameisensäure-äthylester versetzt. Anschliessend wird das Reaktionsgemisch 10 Stunden lang bei 0 bis +5°C weitergerührt, dann filtriert und das Filtrat am Rotationsverdampfer eingeengt. Das Rohprodukt wird an einer Kieselgelsäule mit einer 10:1 Hexan/Methylenchlorid-Mischung chromatographisch gereinigt. Die Titelverbindung der Formel

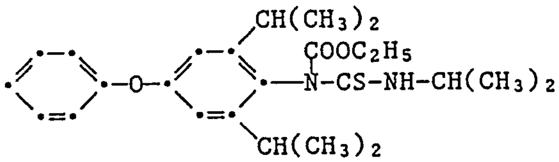

(Verb. 1.3.1.)

liegt in Form von farblosen Kristallen vor; Smp. 107-108°C.

Auf analoge Weise werden die folgenden Verbindungen hergestellt:

Auf analoge Weise werden die folgenden Verbindungen hergestellt:

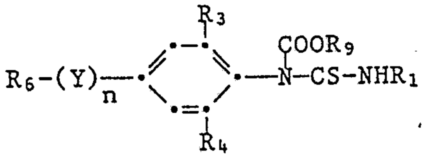

| Verb. Nr. | $R_1$ | $R_9$ | $R_3$ | $R_4$ | $R_6(Y)_n$ | phys. Daten |
|---|---|---|---|---|---|---|
| 1.3.2. | $CH(CH_3)_2$ | $C_2H_5$ | $CH(CH_3)_2$ | $CH(CH_3)_2$ | 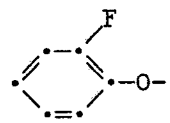 | Smp. 117-119°C |
| 1.3.3. | $CH(CH_3)_2$ | $C_2H_5$ | $CH(CH)_2$ | $CH(CH_3)_2$ | 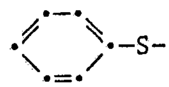 | Smp. 88-90°C |
| 1.3.4. | $CH(CH_3)_2$ | $C_2H_5$ | $CH_3$ | $CH(CH_3)_2$ | 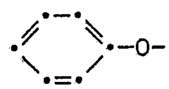 | $n_D^{25}$: 1,5508 |
| 1.3.5. | $CH(CH_3)_2$ | $C_2H_5$ | $C_2H_5$ | $CH(CH_3)C_2H_5$ | 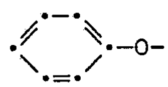 | $n_D^{22}$: 1,5482 |
| 1.3.6. | $CH(CH_3)_2$ | $C_2H_5$ | $C_2H_5$ | $C_2H_5$ | 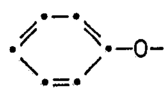 | $n_D^{22}$: 1,5623 |
| 1.3.7. | $CH_2-C(CH_3)_3$ | $C_2H_5$ | $CH(CH_3)_2$ | $CH(CH_3)_2$ | 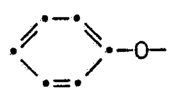 | $n_D^{23}$: 1,5392 |
| 1.3.8. | $CH(CH_3)_2$ | $C_2H_5$ | $CH(CH_3)_2$ | $CH(CH_3)_2$ | 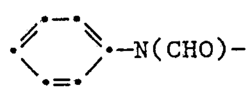 | Smp. 176°C |
| 1.3.9. | $CH(CH_3)_2$ | $C_2H_5$ | $CH(CH_3)_2$ | $CH(CH_3)_2$ | 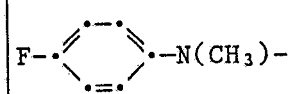 | Smp. 140-142°C |
| 1.3.10. | $CH(CH_3)C_2H_5$ | $C_2H_5$ | $C_2H_5$ | $CH(CH_3)_2$ | 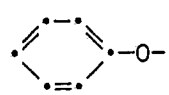 | $n_D^{27}$: 1,5531 |
| | $CH(CH_3)_2$ | $C_2H_5$ | $CH(CH_3)_2$ | $CH(CH_3)_2$ | 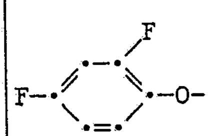 | |
| | $CH(CH_3)_2$ | $CH_3$ | $CH(CH_3)_2$ | $CH(CH_3)_2$ | 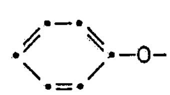 | |
| | $CH(CH_3)_2$ | $C_3H_7$ | $CH(CH_3)_2$ | $CH(CH_3)_2$ | 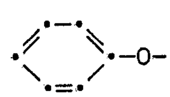 | |

Tabelle Fortsetzung

| Verb. Nr. | $R_1$ | $R_2$ | $R_3$ | $R_4$ | $R_5(Y)_n$ | phys. Daten |
|---|---|---|---|---|---|---|
| | $CH(CH_3)_2$ | $C_4H_9$ | $CH(CH_3)_2$ | $CH(CH_3)_2$ | 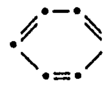 —O— | |
| | $CH(CH_3)_2$ | $C_2H_5$ | $CH(CH_3)_2$ | $CH(CH_3)_2$ | 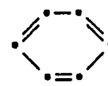 —$CH_2$— | |
| | $CH(CH_3)_2$ | $C_2H_5$ | $C_2H_5$ | $CH(CH_3)_2$ | 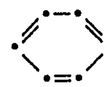 —O— | |
| | $CH(CH_3)_2$ | $C_2H_5$ | $CH(CH_3)_2$ | $CH(CH_3)_2$ | 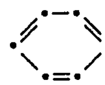 —$N(CH_3)$— | |
| | $C(CH_3)_3$ | $C_2H_5$ | $CH(CH_3)_2$ | $CH(CH_3)_2$ | 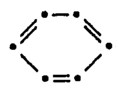 —O— | |
| | $C(CH_3)_3$ | $C_2H_5$ | $CH_3$ | $CH(CH_3)_2$ | 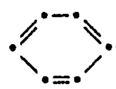 —O— | |

Beispiel 2: Formulierungen von Wirkstoffen der Formeln I und IV gemäss den Herstellungsbeispielen 1.1. und 1.2. und 1.3.
(% = Gewichtsprozent)

2.1. Emulsions-Konzentrate

| | a) | b) |
|---|---|---|
| Wirkstoff gemäss dem Herstellungsbeispiel 1.1. oder 1.2. oder 1.3. | 10 % | 25 % |
| Ca-Dodecyl-benzolsulfonat | - | 5 % |
| Ricinusöl-poly-äthylenglykoläther (36 Mol AeO) | 25 % | 5 % |
| Cyclohexanon | - | 40 % |
| Butanol | 15 % | - |
| Xylolgemisch | - | 25 % |
| Essigester | 50 % | - |

Aus solchen Konzentraten können durch Verdünnen mit Wasser Emulsionen jeder gewünschten Konzentration hergestellt werden.

2.2. Lösungen

| | a) | b) |
|---|---|---|
| Wirkstoff gemäss dem Herstellungsbeispiel 1.1. oder 1.2. oder 1.3. | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190° C) | - | 74 % |

Die Lösungen sind zur Anwendung in Form kleinster Tropfen geeignet.

2.3. Granulate

| | a) | b) |
|---|---|---|
| Wirkstoff gemäss dem Herstellungsbeispiel 1.1. oder 1.2. oder 1.3. | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4. Extruder Granulat

| | |
|---|---|
| Wirkstoff gemäss dem Herstellungsbeispiel 1.1. oder 1.2. oder 1.3. | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5. Umhüllungs-Granulat

| | |
|---|---|
| Wirkstoff gemäss dem Herstellungsbeispiel 1.1. oder 1.2. oder 1.3. | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

2.6. Stäubemittel

| | a) | b) | c) | d) |
|---|---|---|---|---|
| Herstellungsbeispiel 1.1. oder 1.2. oder 1.3. | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

2.7. Spritzpulver

| | a) | b) | c) |
|---|---|---|---|
| Wirkstoff gemäss dem Herstellungsbeispiel 1.1. oder 1.2. oder 1.3. | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

2.8. Suspensions-Konzentrat

| | |
|---|---|
| Wirkstoff gemäss dem Herstellungsbeispiel 1.1. oder 1.2. oder 1.3. | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologische Prüfungen

3.1. Wirkung gegen Schmeissfliegen

Frisch abgelegte Eier der Schmeissfliegenart Lucilia sericata und werden in kleinen Portionen (30-50 Eier) in Reagenzgläser gegeben, in denen zuvor 4 ml Nährmedium mit 1 ml Testlösung, die 80 ppm des zu prüfenden Wirkstoffes enthält, vermischt worden sind. Nach Beimpfung des Kulturmediums werden die Testgläser mit einem Wattestopfen verschlossen und im Brutschrank bei 30°C 4 Tage lang bebrütet. Im unbehandelten Medium entwickeln sich bis zu diesem Zeitpunkt ca. 1 cm lange Larven (Stadium 3). Ist die Substanz aktiv, so sind die Larven zu diesem Zeitpunkt entweder tot oder deutlich zurückgeblieben. Die Auswertung erfolgt nach 96 Stunden; es wird die Mortalität in Prozenten bestimmt.

Verbindungen gemäss den Beispielen 1.1. und 1.2. und 1.3. zeigen in diesem Test gute Wirkung gegen Lucilia sericata.

3.2. Wirkung gegen Aedes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 12,5 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss den Beispielen 1.1. und 1.2. und 1.3. zeigen gute Wirkung in diesem Test.

3.3. Frassgift-Wirkung auf Spodoptera littoralis-Larven ($L_1$)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvalen Stadium besetzt. Der Versuch wird bei 26°C und ca. 50 % relativer Luftfeuchtigkeit durchgeführt. Nach 2 und 3 Tagen werden Mortalität, Entwicklungs- und Häutungsstörungen der angesetzten Larven bestimmt.

Verbindungen gemäss den Beispielen 1.1. und 1.2. und 1.3. zeigen gute Wirkung in diesem Test.

3.4. Wirkung gegen pflanzenschädigende Akarinen: Tetranychus urticae (OP-sensible) und Tetranychus cinnabarinus (OP-tolerant)

Die Primärblätter von Phaseolus vulgaris-Pflanzen werden 16 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt (die Toleranz bezieht sich auf die Verträglichkeit gegenüber Diazinon).

Die so behandelten infestierten Pflanzen werden mit einer Versuchslösung enthaltend 400 ppm der jeweils zu prüfenden Verbindung bis zur Tropfnässe besprüht. Nach 24 Stunden und wiederum nach 7 Tagen werden Imagines und Larven (alle beweglichen Stadien) unter dem Binokular auf lebende und tote Individuen ausgewertet. Man verwendet pro Konzentration und pro Testspezies eine Pflanze. Während des Versuchsverlaufs stehen die Pflanzen in Gewächshauskabinen bei 25°C.

Verbindungen gemäss den Beispielen 1.1. und 1.2. und 1.3. zeigen in diesem Versuch gute Wirkung gegen Tetranychus urticae, und Tetranychus cinnabarinus.

3.5. Kontaktwirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Belichtungsperiode von 16 Stunden durchgeführt.

Verbindungen gemäss den Beispielen 1.1 und 1.2. und 1.3. zeigen in diesem Test gute Wirkung.

3.6. Kontaktwirkung gegen Nilaparvata lugens (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Belichtungsperiode von 16 Stunden durchgeführt.

Verbindungen gemäss den Beispielen 1.1. und 1.2. und 1.3. zeigen in diesem Test gute Wirkung.

3.7. Systemische Wirkung auf Nilaparvata lugens

Etwa 10 Tage alte Reispflanzen (ca. 10 cm hoch) werden in einen Plastik-Becher eingestellt, der 20 ml einer wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wird dann mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im N 2 bis N 3 Stadium besiedelt und mit einem Plastikzylinder abgedeckt. Der Versuch wird bei 20°C und 60 % relativer Luftfeuchtigkeit mit einer Beleuchtungsperiode von 16 Stunden durchgeführt. Nach fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Verbindungen gemäss den Beispielen 1.1. und 1.2. und 1.3. zeigen im obigem Test 80 - 100%ige Wirkung (Mortalität) gegen Nilaparvata lugens.

3.8. Wirkung gegen Bodeninsekten (Diabrotica balteata)

Es werden 350 ml Erde (bestehend aus 95 Vol.-% Sand und 5 Vol.-% Torf) mit jeweils 150 ml von wässrigen Emulsionszubereitungen vermischt, die den zu prüfenden Wirkstoff in einer Konzentration von 400 ppm enthalten. Dann werden Plastikbecher, die einen oberen Durchmeser von ca. 10 cm haben, mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 22°C gehalten. Zehn Tage nach dem Ansatz wird die in den Bechern enthaltene Erde abgesiebt und die zurückbleibenden Larven werden hinsichtlich ihrer Mortalität geprüft.

Verbindungen gemäss den Beispielen 1.1 und 1.2. und 1.3. zeigen gute Wirkung im obigen Test.

Beispiel 3.9. Wirkung gegen Plasmopara viticola auf Reben Residual-protektive Wirkung

Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.

Verbindungen gemäss den Beispielen 1.1. und 1.2. zeigen gegen Plasmopara gute Wirksamkeit. So reduziert z.B. die Verbindung Nr. 1.2.13. den Plasmopara-Befall auf 0 bis 5 %. Unbehandelte aber infizierte Kontrollpflanzen weisen dagegen einen Plasmopara-Befall von 100 % auf.

Beispiel 3.10. Wirkung gegen Dermanyssus gallinae

In einem nach oben offenen Glasbehälter werden 2 bis 3 ml einer 10 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt

und dann kurzfristig umgekehrt, damit die restliche Testlösung von der Watte aufgenommen werden kann. Nach 3 Tagen wird die Mortalität der Milben durch Auszählen der toten Individuen ermittelt und in Prozenten angegeben.

Verbindungen gemäss den Beispielen 1.1. und 1.2. und 1.3. zeigen gute Wirkung im obigen Test.

Beispiel 3.11. Wirkung gegen ektoparasitäre Zecken

5 frische, vollgesogene Boophilus microplus Weibchen werden in einer Reihe dorsal auf einer PVC Platte aufgeklebt und mit einem Wattebausch überdeckt. Danach werden die Testtiere mit 10 ml der wässrigen Testlösung übergossen. Eine Stunde später wird der Wattebausch entfernt und die Zecken über Nacht bei 24°C getrocknet. Nach dem Trocknen werden die Zecken 4 Wochen lang bis zum Ende der Eiablage und Beginn des Larvenschlupfes bis 28°C und 80 % Luftfeuchtigkeit gehalten.

Jede Testsubstanz wird in einer Konzentration von 125 ppm getestet. Die akarizide Wirkung zeigt sich entweder beim Weibchen als Mortalität oder Sterilität oder im Eigelege durch Blockieren der Embryogenese oder des Schlüpfaktes. Alle Substanzen werden gegen zwei verschiedene Zeckenstämme, den OP-resistenten Stamm BIARRA und den Amidin-resistenten Stamm ULAM geprüft.

Die Verbindungen gemäss Beispielen 1.1. und 1.2. und 1.3. zeigen gute Wirkung in obigem Test.

**Patentansprüche**

1. Verbindungen der Formel I

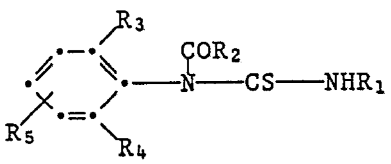

(I),

worin

$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein-oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,

$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes Phenyl oder $C_3$-$C_8$-Cycloalkyl; oder für einen Rest $-OR_9$;

$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;

$R_4$ für $C_1$-$C_{10}$-Alkyl, $C_3$-$C_7$-Cycloalkyl oder $C_5$-$C_6$-Cycloalkenyl;

$R_5$ für Wasserstoff oder $R_6(Y)_n$;

$R_6$ für $C_1$-$C_{10}$-Alkyl, Phenyl, Naphthyl, Indanyl, Tetrahydronaphthyl; oder ein- oder mehrfach duroh Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;

$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;

Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und

n für 0 oder 1

stehen, mit der Massgabe, dass $R_2$ nur dann für den Rest $-OR_9$ stehen darf, wenn $R_5$ eine von Wasserstoff und $C_1$-$C_{10}$-Alkoxy verschiedene Bedeutung hat.

2. Verbindung gemäss Anspruch 1, dadurch gekennzeichnet, dass sie der Formel Ia

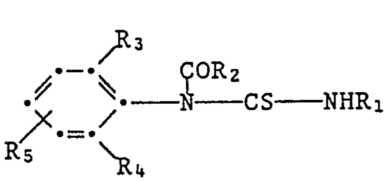

(Ia),

entsprechen, worin

$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl;

$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl;
$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;
$R_4$ für $C_1$-$C_{10}$-Alkyl, $C_3$-$C_7$-Cycloalkyl oder $C_5$-$C_6$-Cycloalkenyl;
$R_5$ für Wasserstoff oder $R_6(Y)_n$;
$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und
n für 0 oder 1
stehen.

3. Verbindungen der Formel Ia gemäss Anspruch 2, worin $R_1$ für $C_1$-$C_5$-Alkyl oder Phenyl-$C_1$-$C_3$-alkyl; $R_2$ für $C_1$-$C_5$-Alkyl, $C_2$-$C_5$-Alkenyl, $C_2$-$C_5$-Alkinyl, Phenyl oder Phenyl-$C_1$-$C_3$-alkyl; $R_3$ für $C_1$-$C_5$-Alkyl, $C_1$-$C_5$-Alkoxy, Cyclopentyl oder Cyclopentenyl; $R_4$ für $C_1$-$C_5$-Alkyl; $R_5$ für Wasserstoff oder $R_6(Y)_n$; $R_6$ für $C_1$-$C_5$-Alkyl, Phenyl, Naphthyl oder Indanyl; oder ein- oder mehrfach durch Fluor, Chlor oder $C_1$-$C_3$-Alkyl substituiertes Phenyl, Naphthyl oder Indanyl; Y für O, S, $SO_2$, NH, N(CHO), $N(CH_3)$ oder $C(R_7)R_8$, wobei $R_7$ und $R_8$ je Wasserstoff oder Methyl bedeuten; und n für 0 oder 1 stehen.

4. Verbindungen der Formel Ia gemäss Anspruch 3, worin $R_1$ für $C_3$-$C_5$-Alkyl oder Phenyl-$C_2$-$C_3$-alkyl; $R_2$ für $C_1$-$C_3$-Alkyl oder Phenyl; $R_3$ für $C_1$-$C_4$-Alkyl, $C_1$-$C_4$-Alkoxy, Cyclopentyl oder Cyclopentenyl; $R_4$ für $C_1$-$C_4$-Alkyl; $R_5$ für Wasserstoff oder $R_6Y$ in 4-Stellung; $R_6$ für $C_1$-$C_4$-Alkyl, Phenyl oder durch 1 oder 2 Fluor substituiertes Phenyl; und Y für O oder S stehen.

5. Die Verbindung gemäss Anspruch 4 der Formeln

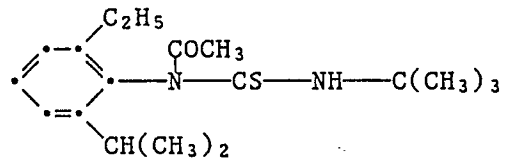
,

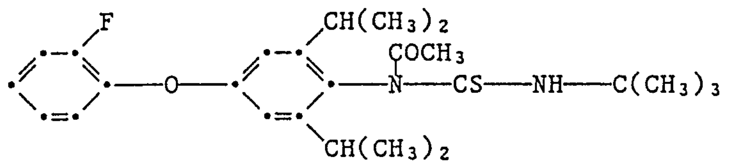
,

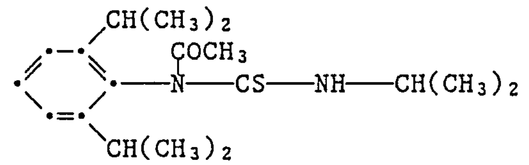
,

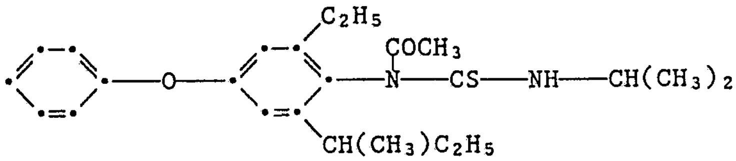
,

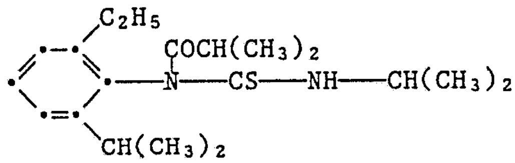
,

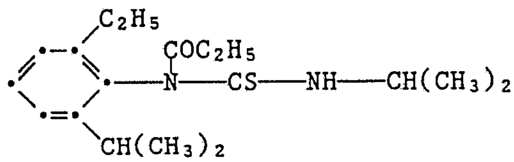
,

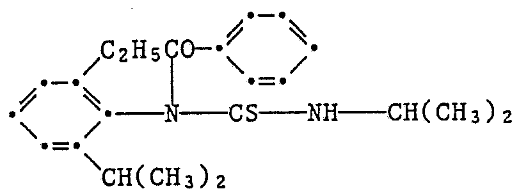
,

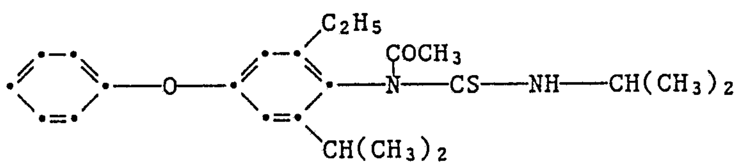
,

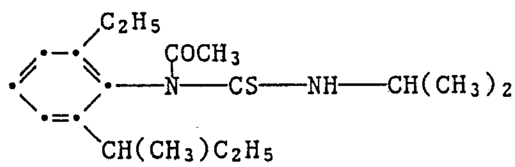
,

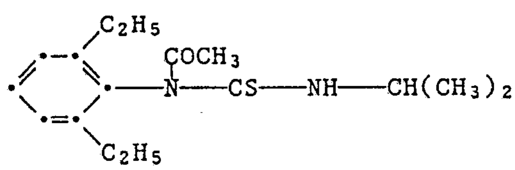
,

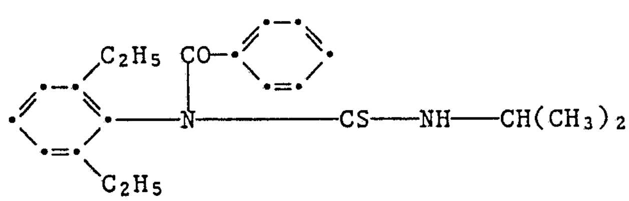
,

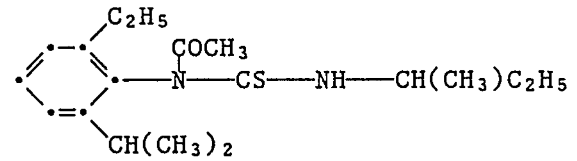

,

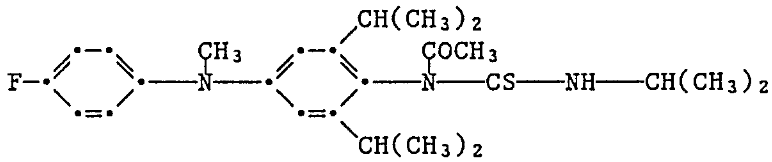

,

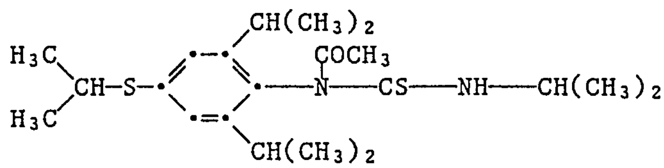

und

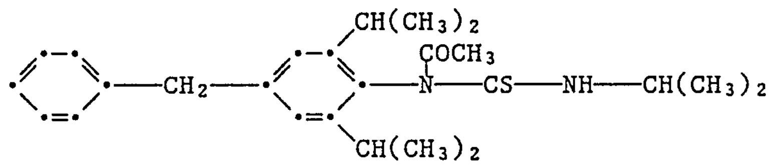

.

6. Verbindungen gemäss Anspruch 1, dadurch gekennzeichnet, dass sie der Formel Ib

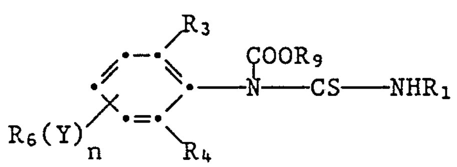

(Ib),

entpsrechen, worin
$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein- oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl;
$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;
$R_4$ für $C_1$-$C_{10}$-Alkyl, $C_3$-$C_7$-Cycloalkyl oder $C_5$-$C_6$-Cycloalkenyl;
$R_6$ für Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;
$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und
n für 0 oder 1
stehen.

7. Verbindungen der Formel Ib gemäss Anspruch 6, worin $R_1$ für $C_1$-$C_5$-Alkyl oder Phenyl-$C_1$-$C_3$-alkyl; $R_3$ für $C_1$-$C_5$-Alkyl, $C_1$-$C_5$-Alkoxy, Cyclopentyl oder Cyclopentenyl; $R_4$ für $C_1$-$C_5$-Alkyl; $R_6$ für Phenyl, Naphthyl oder Indanyl; $R_9$ für $C_1$-$C_5$-Alkyl, $C_3$-$C_5$-Alkenyl, $C_3$-$C_5$-Alkinyl, Phenyl oder Phenyl-$C_1$-$C_3$-alkyl; Y für O, S, $SO_2$, NH, N(CHO) oder $C(R_7)R_8$, wobei $R_7$ und $R_8$ je Wasserstoff oder Methyl bedeuten; und n für 0 oder 1 stehen.

8. Verbindungen der Formel Ib gemäss Anspruch 6, worin $R_1$ für $C_3$-$C_5$-Alkyl oder Phenyl-$C_2$-$C_3$-alkyl; $R_3$ für $C_1$-$C_4$-Alkyl, $C_1$-$C_4$-Alkoxy, oder Cyclopentyl; $R_4$ für $C_1$-$C_4$-Alkyl; $R_6$ für Phenyl oder durch 1 oder 2 Fluor substituiertes Phenyl; $R_9$ für $C_1$-$C_3$-Alkyl oder Phenyl; Y in 4-Stellung für O oder S; und n für 1

stehen.

9. Die Verbindungen gemäss Anspruch 6 der Formeln

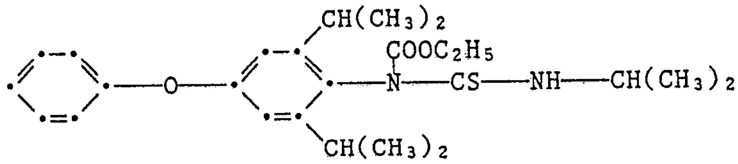

,

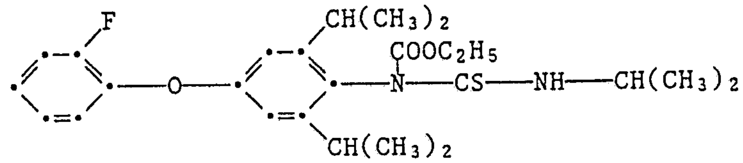

,

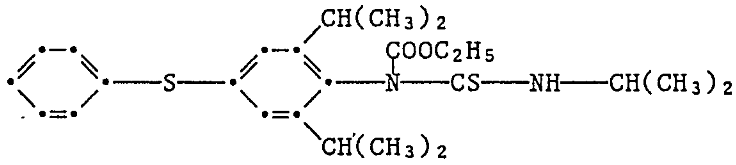

,

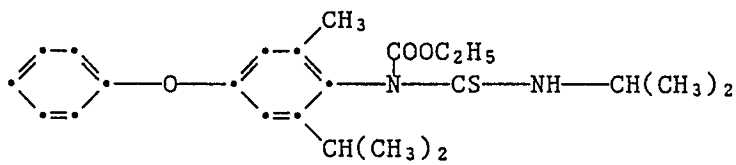

,

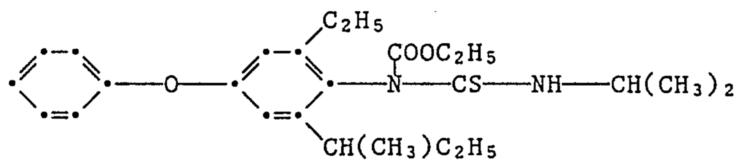

,

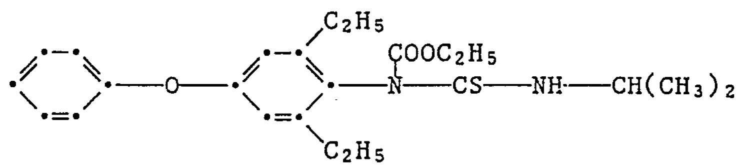

,

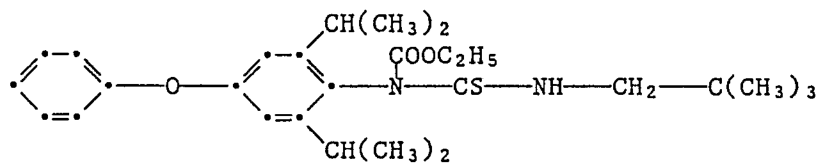

,

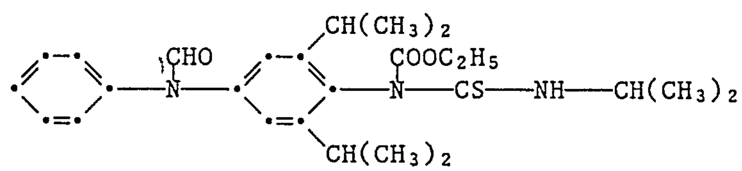

,

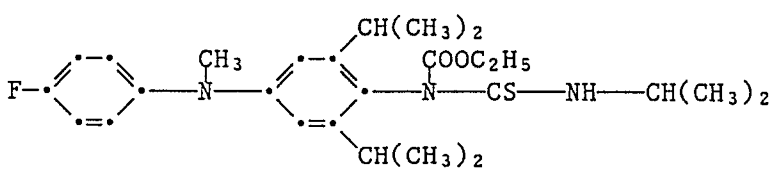

und

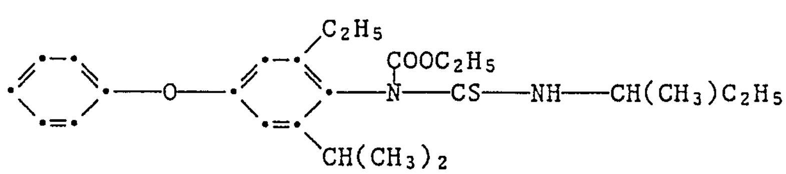

.

10. Verfahren zur Herstellung einer Verbindung der Formel I

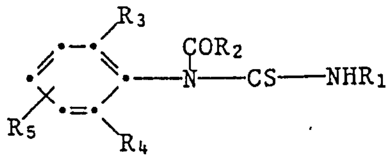

(I),

worin

$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,

$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes Phenyl oder $C_3$-$C_8$-Cycloalkyl; oder für einen Rest -$OR_9$;

$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;

$R_4$ für $C_1$-$C_{10}$-Alkyl, $C_3$-$C_7$-Cycloalkyl oder $C_5$-$C_6$-Cycloalkenyl;

$R_5$ für Wasserstoff oder $R_6(Y)_n$;

$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;

$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;

Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und

n für 0 oder 1

stehen, mit der Massgabe, dass $R_2$ nur dann für den Rest -$OR_9$ stehen darf, wenn $R_5$ eine von Wasserstoff und $C_1$-$C_{10}$-Alkoxy verschiedene Bedeutung hat, dadurch gekennzeichnet, dass man

a) einen Thioharnstoff der Formel II

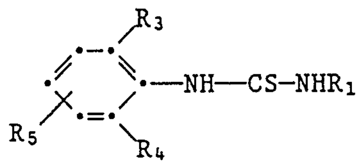

(II)

mit einem Acylhalogenid der Formel III

$XCOR_2$ (III)

in einem Lösungsmittel und in Gegenwart einer Base unter normalem Druck bei -30 bis +100°C umsetzt.

b) ein Salz eines Isothioharnstoffs der Formel IV

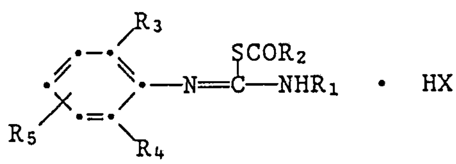

(IV)

bei Raumtemperatur in einer wässrigen Lösung hydrolysiert, wobei in den Formeln II bis IV $R_1$ bis $R_5$ die unter Formel I angegebene Bedeutung haben und X dem Rest einer Halogenwasserstoffsäure entspricht.

11. Schädlingsbekämpfungsmittel, welches als aktive Komponente eine pestizid wirksame Menge mindestens einer Verbindung der Formel I

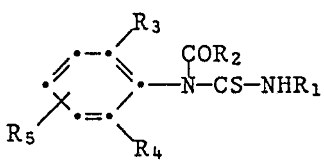

(I)

oder eine Verbindung der Formel IV

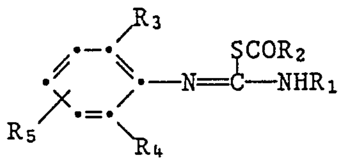 • HX (IV)

enthält, worin
$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein- oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,
$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes Phenyl oder $C_3$-$C_8$-Cycloalkyl; oder für einen Rest -$OR_9$;
$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;
$R_4$ für $C_1$-$C_{10}$-Alkyl, $C_3$-$C_7$-Cycloalkyl oder $C_5$-$C_6$-Cycloalkenyl;
$R_5$ für Wasserstoff oder $R_6(Y)_n$;
$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;
$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und
n für 0 oder 1 stehen und
X den Rest einer Halogenwasserstoffsäure bedeutet, mit der Massgabe, dass $R_2$ nur dann für den Rest -$OR_9$ stehen darf, wenn $R_5$ eine von Wasserstoff und $C_1$-$C_{10}$-Alkoxy verschiedene Bedeutung hat, zusammen mit geeigneten Träger und/oder Zuschlagstoffen.

12. Verwendung einer Verbindung der Formel I

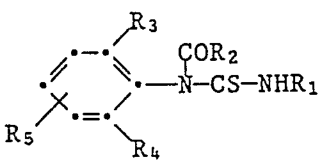 (I)

oder einer Verbindung der Formel IV

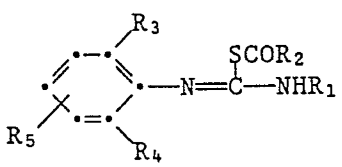 • HX (IV),

worin
$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein- oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,
$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes Phenyl oder $C_3$-$C_8$-Cycloalkyl; oder für einen Rest -$OR_9$;
$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;
$R_4$ für $C_1$-$C_{10}$-Alkyl, $C_3$-$C_7$-Cycloalkyl oder $C_5$-$C_6$-Cycloalkenyl;
$R_5$ für Wasserstoff oder $R_6(Y)_n$;
$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;
$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein-

oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und
n für 0 oder 1 stehen und
X den Rest einer Halogenwasserstoffsäure bedeutet, mit der Massgabe, dass $R_2$ nur dann für den Rest $-OR_9$ stehen darf, wenn $R_5$ eine von Wasserstoff und $C_1$-$C_{10}$-Alkoxy verschiedene Bedeutung hat, zusammen mit geeigneten Träger und/oder Zuschlagstoffen.

13. Verwendung gemäss Anspruch 12 einer Verbindung der Formel I oder IV zur Bekämpfung von Insekten, Arachniden und pflanzenpathogenen Pilzen.

14. Verfahren zur Bekämpfung von Schädlingen an Tieren und Pflanzen, dadurch gekennzeichnet, dass man die Schädlinge in ihren verschiedenen Entwicklungsstadien mit einer Verbindung der Formel I

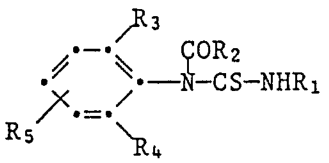

(I)

oder mit einer Verbindung der Formel IV

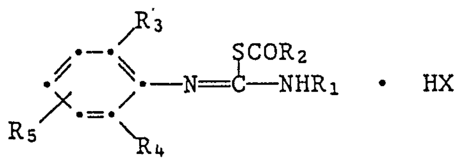

(IV),

worin
$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein- oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,
$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes Phenyl oder $C_3$-$C_8$-Cycloalkyl; oder für einen Rest $-OR_9$;
$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;
$R_4$ für $C_1$-$C_{10}$-Alkyl, $C_3$-$C_7$-Cycloalkyl oder $C_5$-$C_6$-Cycloalkenyl;
$R_5$ für Wasserstoff oder $R_6(Y)_n$;
$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;
$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und
n für 0 oder 1 stehen und
X den Rest einer Halogenwasserstoffsäure bedeutet, mit der Massgabe, dass $R_2$ nur dann für den Rest $-OR_9$ stehen darf, wenn $R_5$ eine von Wasserstoff und $C_1$-$C_{10}$-Alkoxy verschiedene Bedeutung hat.

15. Verbindung der Formel IV

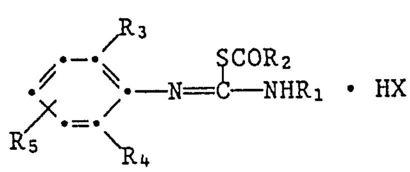

(IV),

worin
$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein- oder mehrfach durch Halogen oder $C_1$-$C_5$-Alkyl substituiertes $C_3$-$C_8$-Cycloalkyl; oder $C_3$-$C_8$-Cycloal-

kyl-$C_1$-$C_4$-alkyl,
$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes Phenyl oder $C_3$-$C_8$-Cycloalkyl; oder für einen Rest -$OR_9$;
$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;
$R_4$ für $C_1$-$C_{10}$-Alkyl, $C_3$-$C_7$-Cycloalkyl oder $C_5$-$C_6$-Cycloalkenyl;
$R_5$ für Wasserstoff oder $R_6(Y)_n$;
$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;
$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und
n für 0 oder 1 stehen und
X den Rest einer Halogenwasserstoffsäure bedeutet, mit der Massgabe, dass $R_2$ nur dann für den Rest -$OR_9$ stehen darf, wenn $R_5$ eine von Wasserstoff und $C_1$-$C_{10}$-Alkoxy verschiedene Bedeutung hat.

16. Verbindung gemäss Anspruch 15, dadurch gekennzeichnet, dass sie der Formel IVa

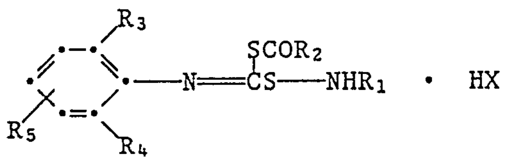

(IVa),

entsprechen, worin
$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl;
$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl, Phenyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl;
$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;
$R_4$ für $C_1$-$C_{10}$-Alkyl, $C_3$-$C_7$-Cycloalkyl oder $C_5$-$C_6$-Cycloalkenyl;
$R_5$ für Wasserstoff oder $R_6(Y)_n$;
$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und n für 0 oder 1 stehen, und
X den Rest einer Halogenwasserstoffsäure bedeutet.

17. Verbindungen der Formel IVa gemäss Anspruch 16, worin $R_1$ für $C_1$-$C_5$-Alkyl oder Phenyl-$C_1$-$C_3$-alkyl; $R_2$ für $C_1$-$C_5$-Alkyl, $C_2$-$C_5$-Alkenyl, $C_2$-$C_5$-Alkinyl, Phenyl oder Phenyl-$C_1$-$C_3$-alkyl; $R_3$ für $C_1$-$C_5$-Alkyl, $C_1$-$C_5$-Alkoxy, Cyclopentyl oder Cyclopentenyl; $R_4$ für $C_1$-$C_5$-Alkyl; $R_5$ für Wasserstoff oder $R_6(Y)_n$; $R_6$ für $C_1$-$C_5$-Alkyl, Phenyl, Naphthyl oder Indanyl; oder ein- oder mehrfach durch Fluor, Chlor oder $C_1$-$C_3$-Alkyl substituiertes Phenyl, Naphthyl oder Indanyl; Y für O, S, $SO_2$, NH, N(CHO), $N(CH_3)$ oder $C(R_7)R_8$, wobei $R_7$ und $R_8$ je Wasserstoff oder Methyl bedeuten; und n für 0 oder 1 stehen; und X den Rest einer Halogenwasserstoffsäure bedeutet.

18. Verbindungen der Formel IVa gemäss Anspruch 17, worin $R_1$ für $C_3$-$C_5$-Alkyl oder Phenyl-$C_2$-$C_3$-alkyl; $R_2$ für $C_1$-$C_3$-Alkyl oder Phenyl; $R_3$ für $C_1$-$C_4$-Alkyl, $C_1$-$C_4$-Alkoxy, Cyclopentyl oder Cyclopentenyl, $R_4$ für $C_1$-$C_4$-Alkyl; $R_5$ für Wasserstoff oder $R_6Y$ in 4-Stellung; $R_6$ für $C_1$-$C_4$-Alkyl, Phenyl oder durch 1 oder 2 Fluor substituiertes Phenyl; und Y für O oder S stehen; und X Chlor bedeutet.

19. Die Verbindungen gemäss Anspruch 18 der Formeln

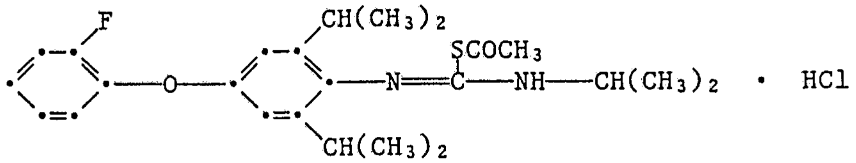 ,

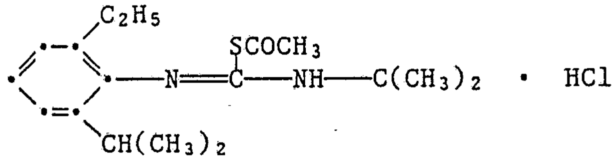 ,

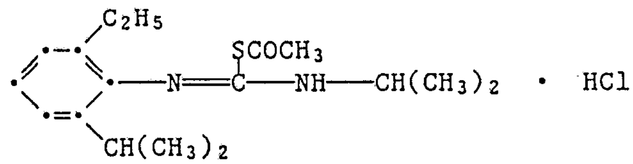 und

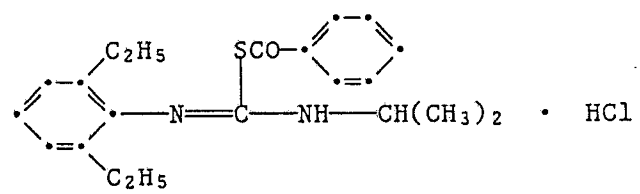 .

20. Verfahren zur Herstellung einer Verbindung der Formel IV

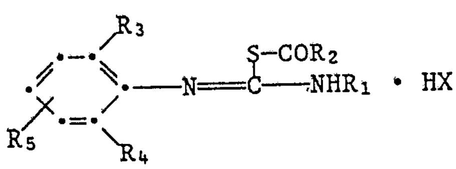 (IV),

worin

$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein- oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,

$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes Phenyl oder $C_3$-$C_8$-Cycloalkyl; oder für einen Rest -$OR_9$;

$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;

$R_4$ für $C_1$-$C_{10}$-Alkyl, $C_3$-$C_7$-Cycloalkyl oder $C_5$-$C_6$-Cycloalkenyl;

$R_5$ für Wasserstoff oder $R_6(Y)_n$;

$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;

$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;

Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und

n für 0 oder 1 stehen und

X den Rest einer Halogenwasserstoffsäure bedeutet, mit der Massgabe, dass $R_2$ nur dann für den Rest -$OR_9$ stehen darf, wenn $R_5$ eine von Wasserstoff und $C_1$-$C_{10}$-Alkoxy verschiedene Bedeutung hat, dadurch gekennzeichnet, dass man einen Thioharnstoff der Formel II

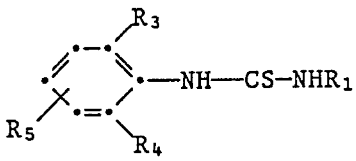

(II)

in einem Lösungsmittel unter normalem Druck bei einer Temperatur von -20 bis +100°C mit einem Acylhalogenid der Formel III

$XCOR_2$ (III)

umsetzt, wobei $R_1$ bis $R_5$ und X die unter Formel IV angegebene Bedeutung haben.

**Patentansprüche für folgenden Vertragsstaat: ES**

1. Verfahren zur Herstellung einer Verbindung der Formel I

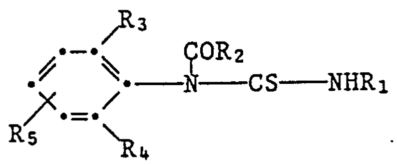

(I),

worin

$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,

$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes Phenyl oder $C_3$-$C_8$-Cycloalkyl; oder für einen Rest -$OR_9$;

$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;

$R_4$ für $C_1$-$C_{10}$-Alkyl, $C_3$-$C_7$-Cycloalkyl oder $C_5$-$C_6$-Cycloalkenyl;

$R_5$ für Wasserstoff oder $R_6(Y)_n$;

$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;

$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;

Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und

n für 0 oder 1

stehen, mit der Massgabe, dass $R_2$ nur dann für den Rest -$OR_9$ stehen darf, wenn $R_5$ eine von Wasserstoff und $C_1$-$C_{10}$-Alkoxy verschiedene Bedeutung hat, dadurch gekennzeichnet, dass man

a) einen Thioharnstoff der Formel II

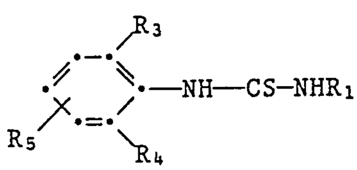

(II)

mit einem Acylhalogenid der Formel III

$XCOR_2$ (III)

in einem Lösungsmittel und in Gegenwart einer Base unter normalem Druck bei -30 bis +100°C umsetzt.

b) ein Salz eines Isothioharnstoffs der Formel IV

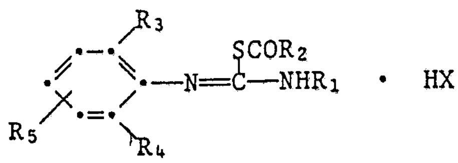

(IV)

bei Raumtemperatur in einer wässrigen Lösung hydrolysiert, wobei in den Formeln II bis IV $R_1$ bis $R_5$ die unter Formel I angegebene Bedeutung haben und X dem Rest einer Halogenwasserstoffsäure entspricht.

2. Verfahren gemäss Anspruch 1 zur Herstellung der Verbindungen der Formel Ia

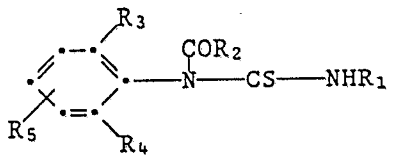

(Ia),

entsprechen, worin

$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl;

$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes $C_3$-$C_8$-Cycloalkyl;

$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;

$R_4$ für $C_1$-$C_{10}$-Alkyl, $C_3$-$C_7$-Cycloalkyl oder $C_5$-$C_6$-Cycloalkenyl;

$R_5$ für Wasserstoff oder $R_6(Y)_n$;

$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;

Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und

n für 0 oder 1

stehen.

3. Verfahren gemäss Anspruch 2, dadurch gekennzeichnet, dass $R_1$ für $C_1$-$C_5$-Alkyl oder Phenyl-$C_1$-$C_3$-alkyl; $R_2$ für $C_1$-$C_5$-Alkyl, $C_2$-$C_5$-Alkenyl, $C_2$-$C_5$-Alkinyl, Phenyl oder Phenyl-$C_1$-$C_3$-alkyl; $R_3$ für $C_1$-$C_5$-Alkyl, $C_1$-$C_5$-Alkoxy, Cyclopentyl oder Cyclopentenyl; $R_4$ für $C_1$-$C_5$-Alkyl; $R_5$ für Wasserstoff oder $R_6(Y)_n$; $R_6$ für $C_1$-$C_5$-Alkyl, Phenyl, Naphthyl oder Indanyl; oder ein- oder mehrfach durch Fluor, Chlor oder $C_1$-$C_3$-Alkyl substituiertes Phenyl, Naphthyl oder Indanyl; Y für O, S, $SO_2$, NH, N(CHO), $N(CH_3)$ oder $C(R_7)R_8$, wobei $R_7$ und $R_8$ je Wasserstoff oder Methyl bedeuten; und n für 0 oder 1 stehen.

4. Verfahren gemäss Anspruch 3, dadurch gekennzeichnet, dass $R_1$ für $C_3$-$C_5$-Alkyl oder Phenyl-$C_2$-$C_3$-alkyl; $R_2$ für $C_1$-$C_3$-Alkyl oder Phenyl; $R_3$ für $C_1$-$C_4$-Alkyl, $C_1$-$C_4$-Alkoxy, Cyclopentyl oder Cyclopentenyl; $R_4$ für $C_1$-$C_4$-Alkyl; $R_5$ für Wasserstoff oder $R_6Y$ in 4-Stellung; $R_6$ für $C_1$-$C_4$-Alkyl, Phenyl oder durch 1 oder 2 Fluor substituiertes Phenyl; und Y für O oder S stehen.

5. Verfahren gemäss Anspruch 4 zur Herstellung der Verbindungen der Formeln

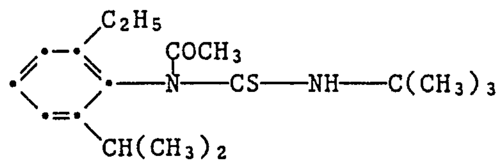

,

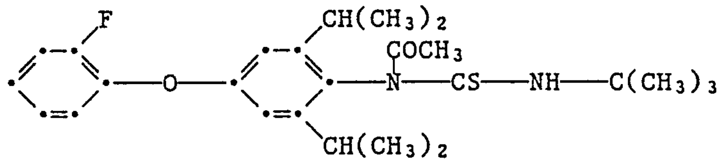

,

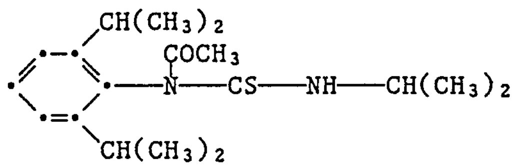

,

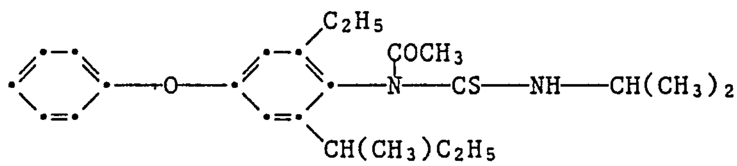

,

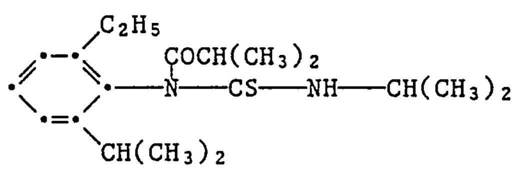

,

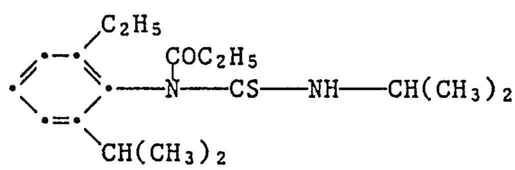

,

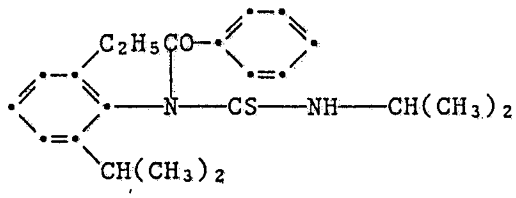

,

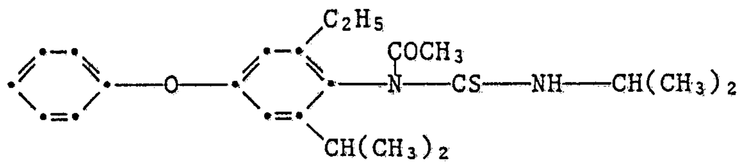

,

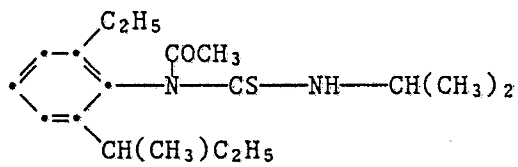

,

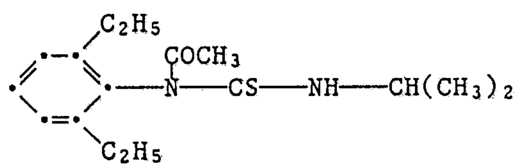

,

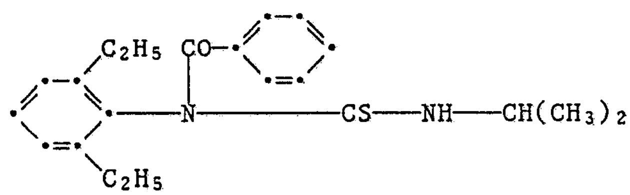

,

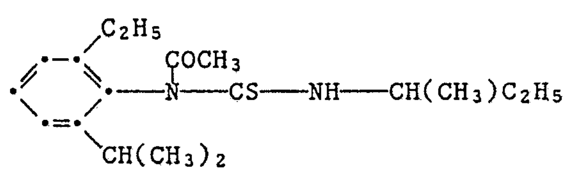

,

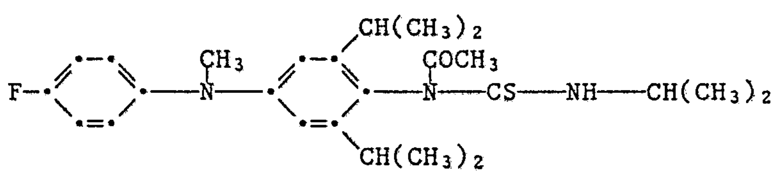

,

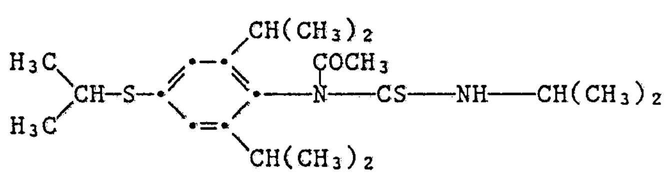

und

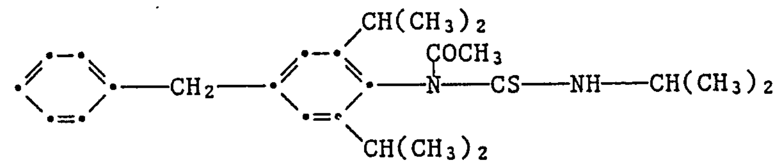

.

6. Verfahren gemäss Anspruch 1 zur Herstellung der Verbindungen der Formel Ib

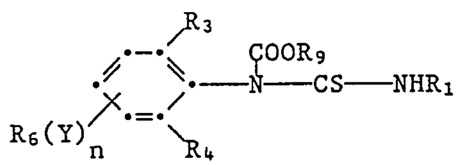

(Ib),

entsprechen, worin

$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein- oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,

$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;

$R_4$ für $C_1$-$C_{10}$-Alkyl, $C_3$-$C_7$-Cycloalkyl oder $C_5$-$C_6$-Cycloalkenyl;

$R_6$ für Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;

$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch Halogen oder $C_1$-$C_5$-Alkyl substituiertes Phenyl oder $C_3$-$C_8$-Cycloalkyl;

Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und

n für 0 oder 1

stehen.

7. Verfahren gemäss Anspruch 6, dadurch gekennzeichnet, dass $R_1$ für $C_1$-$C_5$-Alkyl oder Phenyl-$C_1$-$C_3$-alkyl; $R_3$ für $C_1$-$C_5$-Alkyl, $C_1$-$C_5$-Alkoxy, Cyclopentyl oder Cyclopentenyl; $R_4$ für $C_1$-$C_5$-Alkyl; $R_6$ für Phenyl, Naphthyl oder Indanyl; $R_9$ für $C_1$-$C_5$-Alkyl, $C_3$-$C_5$-Alkenyl, $C_3$-$C_5$-Alkinyl, Phenyl oder Phenyl-$C_1$-$C_3$-alkyl; Y für O, S, $SO_2$, NH, N(CHO) oder $C(R_7)R_8$, wobei $R_7$ und $R_8$ je Wasserstoff oder Methyl bedeuten; und n für 0 oder 1 stehen.

8. Verfahren gemäss Anspruch 6, dadurch gekennzeichnet, dass $R_1$ für $C_3$-$C_5$-Alkyl oder Phenyl-$C_2$-$C_3$-alkyl; $R_3$ für $C_1$-$C_4$-Alkyl, $C_1$-$C_4$-Alkoxy, oder Cyclopentyl; $R_4$ für $C_1$-$C_4$-Alkyl; $R_6$ für Phenyl oder durch 1 oder 2 Fluor substituiertes Phenyl; $R_9$ für $C_1$-$C_3$-Alkyl oder Phenyl; Y in 4-Stellung für O oder S; und n für 1 stehen.

9. Verfahren gemäss Anspruch 6 zur Herstellung der Verbindungen der Formeln

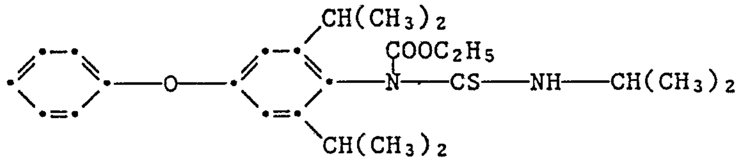

,

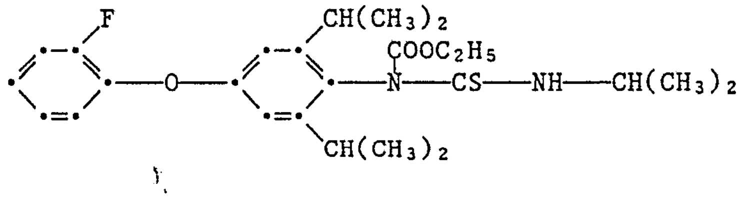

,

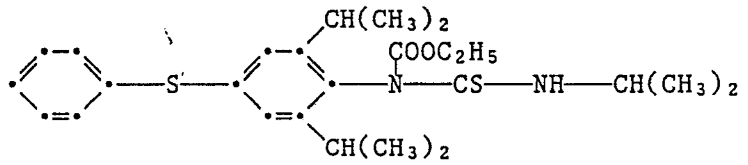

,

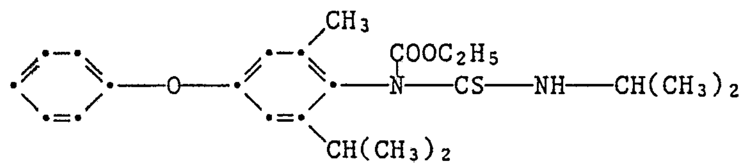

,

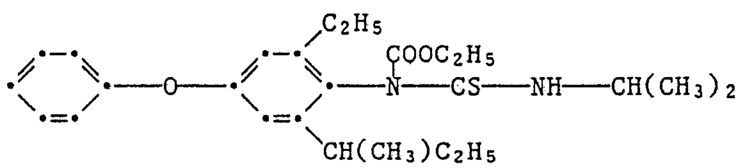

,

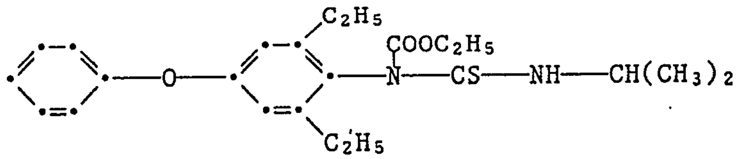

,

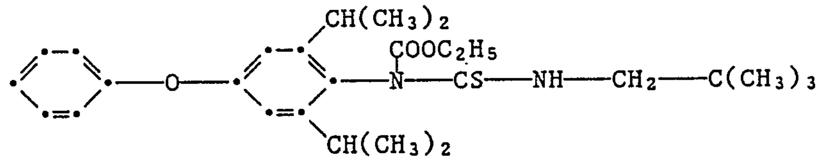

,

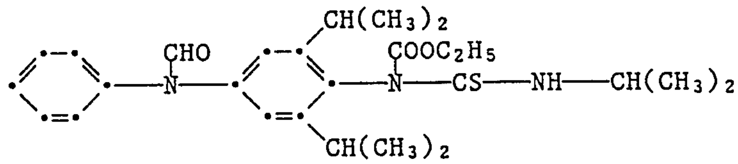

,

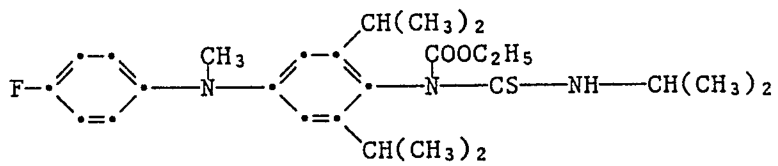

und

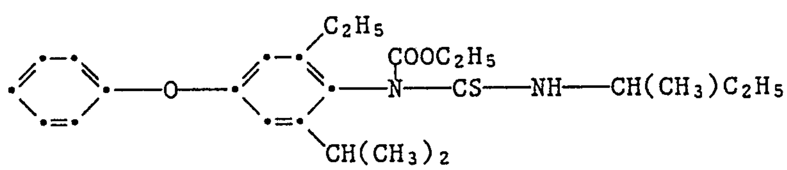

.

10. Verfahren zur Herstellung einer Verbindung der Formel IV

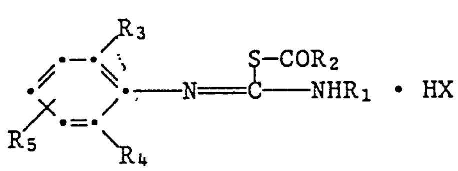

(IV),

worin

$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; ein- oder mehrfach durch Halogen oder $C_1$-$C_5$-Alkyl substituiertes $C_3$-$C_8$-Cycloalkyl; oder $C_3$-$C_8$-Cycloalkyl-$C_1$-$C_4$-alkyl,

$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl oder Phenyl-$C_1$-$C_7$-alkyl; $C_3$-$C_8$-Cycloalkyl; ein- oder mehrfach durch Halogen oder $C_1$-$C_5$-Alkyl substituiertes Phenyl oder $C_3$-$C_8$-Cycloalkyl; oder für einen Rest -$OR_9$;

$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;

$R_4$ für $C_1$-$C_{10}$-Alkyl, $C_3$-$C_7$-Cycloalkyl oder $C_5$-$C_6$-Cycloalkenyl;

$R_5$ für Wasserstoff oder $R_6(Y)_n$;

$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;

$R_9$ für $C_1$-$C_{10}$-Alkyl, $C_3$-$C_{10}$-Alkenyl, $C_3$-$C_{10}$-Alkinyl, Phenyl, Phenyl-$C_1$-$C_7$-alkyl, $C_3$-$C_8$-Cycloalkyl; ein- oder mehrfach halogeniertes oder ein- oder mehrfach durch Sauerstoff und/oder Schwefel unterbrochenes $C_1$-$C_{10}$-Alkyl, $C_3$-$C_{10}$-Alkenyl, $C_3$-$C_{10}$-Alkinyl, Phenyl-$C_1$-$C_7$-alkyl; oder ein- oder mehrfach durch

Halogen oder $C_1$-$C_5$-Alkyl substituiertes Phenyl oder $C_3$-$C_8$-Cycloalkyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und
n für 0 oder 1 stehen und
X den Rest einer Halogenwasserstoffsäure bedeutet, mit der Massgabe, dass $R_2$ nur dann für den Rest $-OR_9$ stehen darf, wenn $R_5$ eine von Wasserstoff und $C_1$-$C_{10}$-Alkoxy verschiedene Bedeutung hat, dadurch gekennzeichnet, dass man einen Thioharnstoff der Formel II

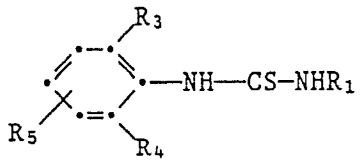

(II)

in einem Lösungsmittel unter normalem Druck bei einer Temperatur von -20 bis +100°C mit einem Acylhalogenid der Formel III
$XCOR_2$ (III)
umsetzt, wobei $R_1$ bis $R_5$ und X die unter Formel IV angegebene Bedeutung haben.

11. Verfahren gemäss Anspruch 10 zur Herstellung der Verbindungen der Formel IVa

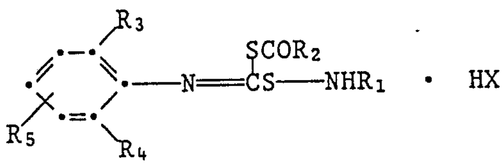

(IVa),

entsprechen, worin
$R_1$ für $C_1$-$C_{10}$-Alkyl, Phenyl-$C_1$-$C_7$-alkyl; ein- oder mehrfach halogeniertes oder ein- oder mehrfach durch Sauerstoff und/oder Schwefel unterbrochenes $C_1$-$C_{10}$-Alkyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl;
$R_2$ für $C_1$-$C_{10}$-Alkyl, $C_2$-$C_{10}$-Alkenyl, $C_2$-$C_{10}$-Alkinyl, Phenyl, Phenyl-$C_1$-$C_7$-alkyl, ein- oder mehrfach halogeniertes oder ein- oder mehrfach durch Sauerstoff und/oder Schwefel unterbrochenes $C_1$-$C_{10}$-Alkyl, $C_2$-$C_{10}$-Alkenyl, $C_2$-$C_{10}$-Alkinyl, Phenyl oder Phenyl-$C_1$-$C_7$-alkyl; $C_3$-$C_8$-Cycloalkyl; oder ein- oder mehrfach durch Halogen oder $C_1$-$C_5$-Alkyl substituiertes $C_3$-$C_8$-Cycloalkyl;
$R_3$ für $C_1$-$C_{10}$-Alkyl, $C_1$-$C_{10}$-Alkoxy, $C_3$-$C_7$-Cycloalkyl oder $C_5$-$C_6$-Cycloalkenyl;
$R_4$ für $C_1$-$C_{10}$-Alkyl, $C_3$-$C_7$-Cycloalkyl oder $C_5$-$C_6$-Cycloalkenyl;
$R_5$ für Wasserstoff oder $R_6(Y)_n$;
$R_6$ für $C_1$-$C_{10}$-Alkyl, Phenyl, Naphthyl, Indanyl, Tetrahydronaphthyl; oder ein- oder mehrfach durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl, Naphthyl, Indanyl oder Tetrahydronaphthyl;
Y für O, S, SO, $SO_2$, NH, N(CHO), $N(CH_3)$ oder $C(R_7)R_8$, wobei $R_7$ und $R_8$ je Wasserstoff oder $C_1$-$C_4$-Alkyl bedeuten; und n für 0 oder 1 stehen, und
X den Rest einer Halogenwasserstoffsäure bedeutet.

12. Verfahren gemäss Anspruch 11, dadurch gekennzeichnet, dass $R_1$ für $C_1$-$C_5$-Alkyl oder Phenyl-$C_1$-$C_3$-alkyl; $R_2$ für $C_1$-$C_5$-Alkyl, $C_2$-$C_5$-Alkenyl, $C_2$-$C_5$-Alkinyl, Phenyl oder Phenyl-$C_1$-$C_3$-alkyl; $R_3$ für $C_1$-$C_5$-Alkyl, $C_1$-$C_5$-Alkoxy, Cyclopentyl oder Cyclopentenyl; $R_4$ für $C_1$-$C_5$-Alkyl; $R_5$ für Wasserstoff oder $R_6(Y)_n$; $R_6$ für $C_1$-$C_5$-Alkyl, Phenyl, Naphthyl oder Indanyl; oder ein- oder mehrfach durch Fluor, Chlor oder $C_1$-$C_3$-Alkyl substituiertes Phenyl, Naphthyl oder Indanyl; Y für O, S, $SO_2$, NH, N(CHO), $N(CH_3)$ oder $C(R_7)R_8$, wobei $R_7$ und $R_8$ je Wasserstoff oder Methyl bedeuten; und n für 0 oder 1 stehen; und X den Rest einer Halogenwasserstoffsäure bedeutet.

13. Verfahren gemäss Anspruch 12, dadurch gekennzeichnet, dass $R_1$ für $C_3$-$C_5$-Alkyl oder Phenyl-$C_2$-$C_3$-alkyl; $R_2$ für $C_1$-$C_3$-Alkyl oder Phenyl; $R_3$ für $C_1$-$C_4$-Alkyl, $C_1$-$C_4$-Alkoxy, Cyclopentyl oder Cyclopentenyl; $R_4$ für $C_1$-$C_4$-Alkyl; $R_5$ für Wasserstoff oder $R_6Y$ in 4-Stellung; $R_6$ für $C_1$-$C_4$-Alkyl, Phenyl oder durch 1 oder 2 Fluor substituiertes Phenyl; und Y für O oder S stehen; und X Chlor bedeutet.

14. Verfahren gemäss Anspruch 13 zur Herstellung der Verbindungen der Formeln

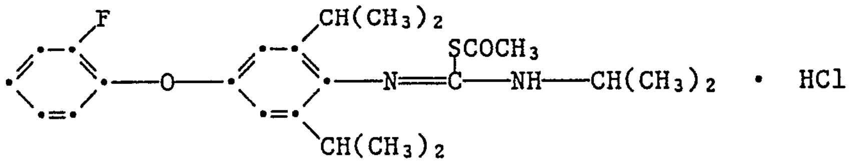

,

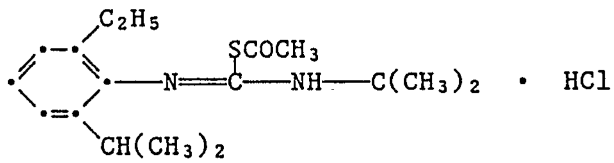

,

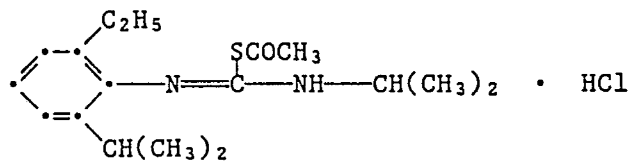

und

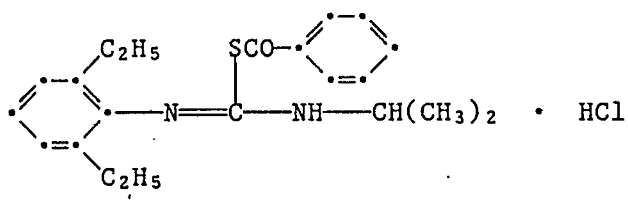

.